# EUROPEAN PATENT APPLICATION

(11) **EP 1 889 613 A1**
(43) Date of publication of application: **20.02.2008**
(21) Application number: 06756505.1
(22) Date of filing: 23.05.2006
(51) Int. Cl.: A61K 31/122, A61K 31/192, A61K 31/381, A61K 31/42, A61K 31/426, A61K 31/4427, A61K 31/517, A61K 45/00, A61P 3/06, A61P 21/00

(54) **PHARMACEUTICAL COMPOSITION COMPRISING VITAMIN K**

(30) Priority: 27.05.2005 JP 2005155837
(71) Applicant: Shionogi & Co., Ltd., Osaka Osaka 541-0045 (JP)
(72) Inventor: INOUE, Satoshi, Toyonaka-shi, Osaka 5610825 (JP); SATO, Seiji, Toyonaka-shi, Osaka 5610825 (JP); TORII, Mikinori, Toyonaka-shi, Osaka 5610825 (JP); SUGITA, Ken-ichi, Fukushima-ku, Osaka-shi, Osaka 5530002 (JP); KYOLAWA, Yoshimasa, Toyonaka-shi, Osaka 5610825 (JP)
(74) Representative: HOFFMANN EITLE
(86) International application number: PCT/JP2006/310249
(87) International publication number: WO 2006/126541

(57) **Abstract**

It is found that compounds having PPARδ agonistic activity induced abnormal blood coagulation or muscular disorder. A pharmaceutical combination comprising vitamin K and a compound having PPARδ agonistic activity can prevent the abnormal blood coagulation. A pharmaceutical composition comprising vitamin K can prevent muscular disorder.

## Description

### Field of the Invention

The present invention relates to a pharmaceutical combination comprising vitamin K, a compound which has peroxisome proliferator-activated receptor (hereinafter referred to as PPAR) δ agonistic activity and which is useful as a medicine, and/or statin compound.

### Background Art

Peroxisome proliferators which proliferate an intracellular granule, peroxisome, are thought as important controlling elements of lipid metabolism. A nuclear receptor, PPAR, which is activated by the peroxisome proliferator has turned out to be a multifunctional receptor concerning incretion, metabolism, inflammation or the like. Therefore, the ligand is thought to be able to apply as various medicines and the number of researches is recently increasing.

The subtype genes of PPARs are found from various animal organs and formed a family. In mammals, PPARs are classified into three subtypes of PPARα, PPARδ (also referred to as PPARβ) and PPARγ.

It was reported that transgenic mice in which PPARδ was overexpressed specifically in adipocyte were difficult to become obese, hyperlipemia or the like (Non-patent Document 1). Therefore, PPARδ agonists can be used as an antiobestic drug, therapeutic agent for hyperlipemia or antidiabetic drug. Additionally, PPARδ agonists are suggested the possibility as therapeutic agents for colonic cancer, osteoporosis, sterility, psoriasis, multiple sclerosis or the like. Various compounds such as GW501516 (Patent Document 1) have been disclosed as a compound having PPARδ agonistic activity. Patent Document 1 discloses that, for example, GW501516 showed HDL-raising and suggests that PPARδ agonists are also useful as a HDL-raising agent. However, no document discloses that these compounds having PPARδ agonistic activity cause abnormal blood coagulation or muscular disorder.

On the other hand, there are some medications which cause tendency to bleed like some antibiotics. Various factors are involved with blood coagulation. It is known that examples of a cause of abnormal blood coagulation are decreased blood platelet function, coagulating system abnormalities related to vitamin K or the like.

"Coagulating system abnormalities related to vitamin K" include deficiency symptom of vitamin K. They are the abnormalities involved with a vitamin K cycle and γ-glutamyl carboxylase which are necessary when prothrombin and blood coagulation factors (VII, IX, X or the like) are produced in liver.

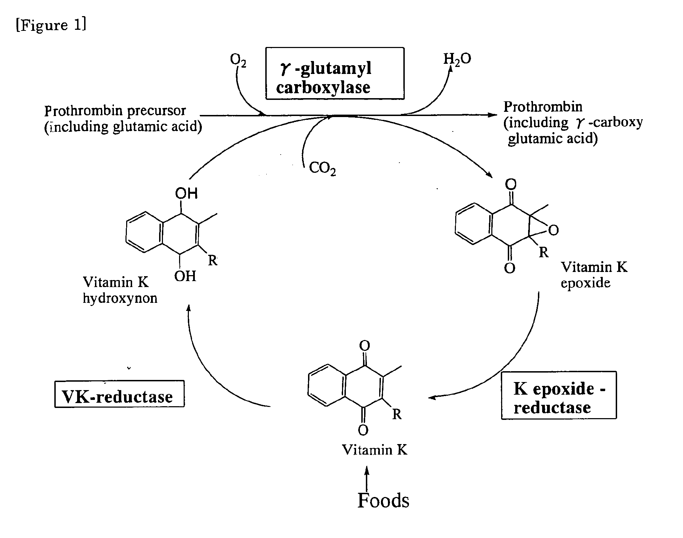

In normal human liver, vitamin K is taken from foods and then reduced by K-reductase to produce vitamin K hydroxynon as described in Figure 1. Vitamin K hydroxynon takes in oxygen to become peroxide, the peroxide conjugates with dehydrogenation reaction of y position of glutamic acid to become vitamin K epoxide and the vitamin K epoxide is reduced by K epoxide-reductase back to vitamin. The generated vitamin is reduced again by K-reductase and reused. This is a vitamin K cycle.

γ-glutamyl carboxylase is an enzyme to transform specific glutamic acid residues in blood coagulation factors, blood coagulation control factors or the like into γ-carboxyglutamic acid under the presence of vitamin K and conjugates with a dehydrogenation reaction in a process to produce vitamin K epoxide from peroxide in a vitamin K cycle.

Therefore, when vitamin K is deficient, when activated vitamin K hydroxynon is not produced because K-reductase is inhibited, when vitamin K can not be reused because the reduction reaction of K epoxide by K epoxide-reductase is inhibited, when γ-glutamyl carboxylase is inhibited or the like, production of γ-carboxyglutamic acid is interrupted, and then vitamin K-dependent blood coagulation factor becomes not to be biosynthesized. That is regarded as a cause of abnormal blood coagulation.

It is suggested that antibiotics such as NMTT in well-known medicines inhibit K epoxide-reductase, and then reuse of vitamin K in a vitamin K cycle is blocked and vitamin K deficiency becomes worse (Non-patent Document 2). In this case, if vitamin K is provided, abnormal blood coagulation is recovered without any trouble in biosynthesis of blood coagulation factors because K-reductase and γ-glutamyl carboxylase are not inhibited. Additionally, it is known that Warfarin (Eisai Co., Ltd.) known as an anticoagulant inhibits not only K epoxide-reductase but also K-reductase. Medicines having the mechanism as well as that of Warfarin are vitamin K antagonists, and abnormal blood coagulation caused by the medicines is difficult to be recovered even if vitamin K is provided. As the above, it is known that the mechanisms and preventive measures against side effect of abnormal blood coagulation are different depending on the medicine, even if a cause of abnormal blood coagulation is "coagulating system abnormalities related to vitamin K".

Additionally, there are medicines which cause muscular disorder (CPK elevation, myopathy, rhabdomyolysis or the like) such as statin drugs (HMG-CoA reductase inhibitors).

The clinical symptoms of muscular disorder are weakness of the extremities (general sick feeling), numbness, muscle pain, muscle weakness, rigidity, swelling, dark reddish-brown urine or the like. In the laboratory findings, acute elevation of myoglobin and muscular escaped enzymes such as CPK, GOT, GPT, LDH or aldolase in blood and myodegeneration are recognized.

Examples of the cause are exogenous (crash syndrome), excessive muscular activity, muscular ischemia, metabolic disorder, drugs, toxicosis, infectious diseases, hereditary diseases and heatstroke. Well-known examples of the causative drug are antipsychotic drugs such as haloperidol and diazepam, statin drugs such as pravastatin, simvastatin, lovastatin and fulvastatin, fibrate drugs such as clofibrate and bezafibrate, antihyperlipidemic drugs such as nicotinic acid derivatives, alcohol, barbiturate and heroin. Though statin drugs in the above drugs are cholesterol synthesis inhibitors and often used because they have strong therapeutic effects on hyperlipidemia, they are known to induce rhabdomyolysis as a side-effect. Rhabdomyolysis or elevation of CPK in blood is known as a side-effect of Haloperidol or fibrate drugs such as clofibrate and bezafibrate. Furthermore, it is reported that combination of a statin drug and fibrate drug, nicotinic acid or immunosuppressant such as cyclosporine caused the increase of frequency of side-effects such as rhabdomyolysis or elevation of CPK in blood and it becomes a clinical problem.

Considering the above cause of disease and clinical condition, a therapy used for muscular disorder is that the causal factors are removed, the patient is kept at rest, and muscular escaped enzymes in blood is naturally decreased by hemodialysis or administering infusion. As it is known that the therapy as above takes a long time, has risk of transformation to acute renal failure for a patient with severe rhabdomyolysis and causes acute recurrent rhabdomyolysis, an aggressive therapy for shorter period is desired. Furthermore, when the cause of muscular disorder is a side-effect by the other medicine, removement of the causal factors equals to interruption of the administeration of the medicine. There is possibility that the other disease progresses in the meantime or the medicine can not be readministerd even after recovering from the symptom of muscular disorder, and the situation has disadvantage for the patient.

No document discloses the role of vitamin K in muscular disorder.
[Patent Document 1] WO01/00603
[Non-patent Document 1] Wang, Y. X. et al., Cell, Vol. 113, p. 159-170 (2003)
[Non-patent Document 2] Kiyohisa Uchida, Kansensho, Vol. 15, no. 5, p. 161-168 (1985)

### Disclosure of Invention

### Problems to be solved by the Invention

The object of the present invention is to provide useful pharmaceutical compositions comprising vitamin K.

### Means for Solving the Problem

The present inventors have intensively studied to find abnormal blood coagulation in rats administered compounds having PPARδ agonistic activity. In these rats, PT (Prothrombin time) and APTT (Activated partial thromboplastin time) were prolonged, activities of vitamin K-dependent coagulation factors were shown as low levels, and vitamin K deficiency symptom (hypoprothrombinaemia) was confirmed. Coagulating system abnormalities related to vitamin K was suggested as the cause. It was thought that, in the coagulating system relating vitamin K, production of γ-carboxyglutamic acid had a trouble by 1 or several cause(s) selected from the following (I) to (IV) and vitamin K-dependent blood coagulation factor(s) could not be biosynthesized.
(I) vitamin K is deficient.
(II) K-reductase is inhibited.
(III) K epoxide-reductase is inhibited.
(IV) γ-glutamyl carboxylase is inhibited.
   When (III) K epoxide-reductase is inhibited and the other causes do not relate, and when (I) vitamin K is deficient, the recovery can be expected by administering vitamin K. However, when the other cause(s) relate, the symptom is not effectively recovered even if by vitamin K is administered.
   The present inventors found that by administering the combination of a compound having PPARδ agonistic activity and vitamin K, the PT and APTT values and the activity values of blood coagulation factors (II, VII, IX and X factors) showed approximate equivalent to those in the case without administeration of a compound having PPARδ agonistic activity.

Furthermore, the present inventors have intensively studied to find muscular disorder in rats administered compounds having PPARδ agonistic activity. In these rats, necrosis and regeneration of skeletal muscle (thigh muscle and soleus muscle) and mononuclear cell-based inflammatory cell infiltration were shown. The present inventors found that by administering the combination of a compound having PPARδ agonistic activity and vitamin K, muscular disorder was not shown or the degree of muscular disorder became mild.

The present invention is the followings.
(1) A pharmaceutical combination comprising
   1) vitamin K, and
   2) a compound having PPARδ agonistic activity and/or statin compound.
(2) The pharmaceutical combination of (1), wherein the vitamin K is at least one or two selected from the group consisting of vitamin K1, vitamin K2 and vitamin K3.
(3) The pharmaceutical combination of (1), wherein the pharmaceutical combination is a combination preparation.
(4) The pharmaceutical combination of (1), wherein the pharmaceutical combination is a kit comprising;
   an agent comprising vitamin K, and
   an agent comprising a compound having PPARδ agonistic activity and/or statin compound.
(5) The pharmaceutical combination of (1), which is a HDL enhancer.
(6) The pharmaceutical combination of (1), which is an antihyperlipidemic drug.
(7) The pharmaceutical combination of (1), which is a muscular disorder suppressant.
(8) The pharmaceutical combination of any one of (1) to (7), wherein the compound having PPARδ agonistic activity is a compound of the formula (I): 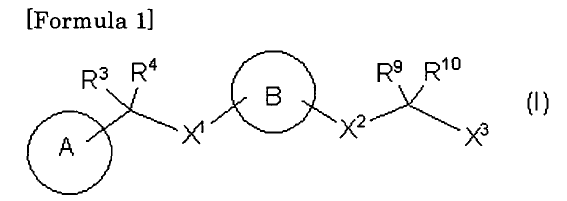 pharmaceutically acceptable salt or solvate thereof,
   wherein
   Ring A is optionally substituted heteroaryl,
   Ring B is optionally substituted aryl or optionally substituted heteroaryl,
   R³ and R⁴ are each independently hydrogen, halogen, optionally substituted lower alkyl, optionally substituted lower alkenyl, optionally substituted lower alkynyl, optionally substituted aryl or optionally substituted heterocycle,
   R⁹ and R¹⁰ are each independently hydrogen, halogen, cyano, optionally substituted lower alkyl, optionally substituted lower alkoxy, optionally substituted amino or optionally substituted aryl,
   X¹ is -O-, -S-, -NR¹¹- wherein R¹¹ is hydrogen, optionally substituted lower alkyl, optionally substituted acyl, optionally substituted lower alkylsulfonyl or optionally substituted arylsulfonyl, -CR¹²R¹³CO-, -(CR¹²R¹³)mO-, -(CR¹²R¹³)mS- or -O(CR¹²R¹³)m-
   wherein R¹² and R¹³ are each independently hydrogen or optionally substituted lower alkyl and m is an integer between 1 and 3, -ON=CR¹⁴ - wherein R¹⁴ is hydrogen or optionally substituted lower alkyl, or a group of the formula: 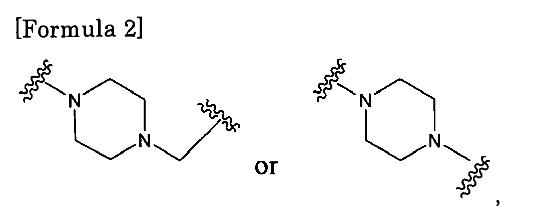 X² is a bond, -O-, -S-, -SO-, -SO₂-, -CR²⁶=CR²⁷- wherein R²⁶ and R²⁷ are each independently hydrogen or optionally substituted lower alkyl, -NR¹⁴- wherein R¹⁴ is hydrogen, optionally substituted lower alkyl, optionally substituted acyl, optionally substituted lower alkylsulfonyl or optionally substituted arylsulfonyl, -CR¹⁵R¹⁶-
   wherein R¹⁵ and R¹⁶ are each independently hydrogen or optionally substituted lower alkyl or -COCR²⁴R²⁵- wherein R²⁴ and R²⁵ are each independently hydrogen or optionally substituted lower alkyl,
   X³ is COOR¹⁷, C(=NR¹⁷)NR¹⁸OR¹⁹ or a group of the formula^{:} 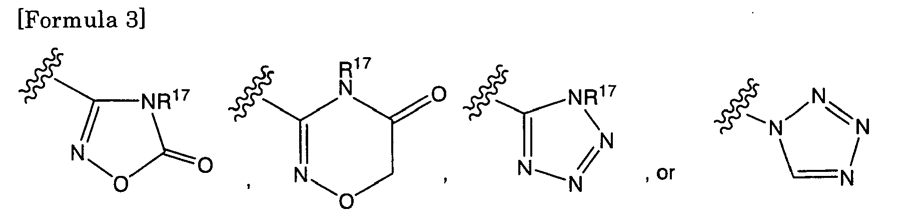 wherein R¹⁷ to R¹⁹ are each independently hydrogen or optionally substituted lower alkyl,
   provided that
   R⁹ and R¹⁶ can be joined together to form a bond,
   R⁹ and R¹⁰ can be taken together to form a ring,
   R⁹ and R²⁵ can be joined together to form a bond,
   R⁹, R¹⁰ and R¹⁵ can be taken together with the neighboring carbon atom to form a ring,
   R¹⁰ and R¹⁵ can be joined together to form a bond, and
   R¹⁰ and R¹⁵ can be taken together with the neighboring carbon atom to form a ring.
(9) The pharmaceutical combination of any one of (1) to (7), wherein the compound having PPARδ agonistic activity is a compound of the formula (II): 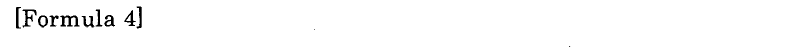 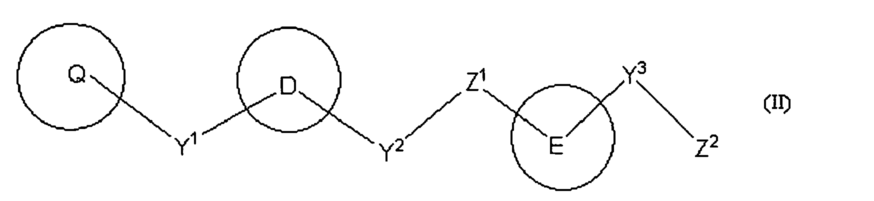 pharmaceutically acceptable salt or solvate thereof,
   wherein,
   Ring Q is monocyclic aryl substituted with at least one of R^{b} and optionally substituted with other group(s), monocyclic heteroaryl substituted with at least one of R^{b} and optionally substituted with other group(s) wherein each R^{b} is optionally substituted aryl, optionally substituted aralkyl , optionally substituted aryloxy, optionally substituted arylthio, optionally substituted heteroaryl, optionally substituted heteroaralkyl, optionally substituted heteroaryloxy or optionally substituted heteroarylthio, substituted fused aryl or substituted fused heteroaryl,
   Y¹ is a bond or -NR^{f}- wherein R^{f} is hydrogen or optionally substituted lower alkyl, Ring D is optionally substituted nonaromatic heterocyclediyl, provided that Ring Q binds with a nitrogen atom of Ring D when Y¹ is a bond,
   a group of the formula: -Y² Z¹ - is a group of the formula: 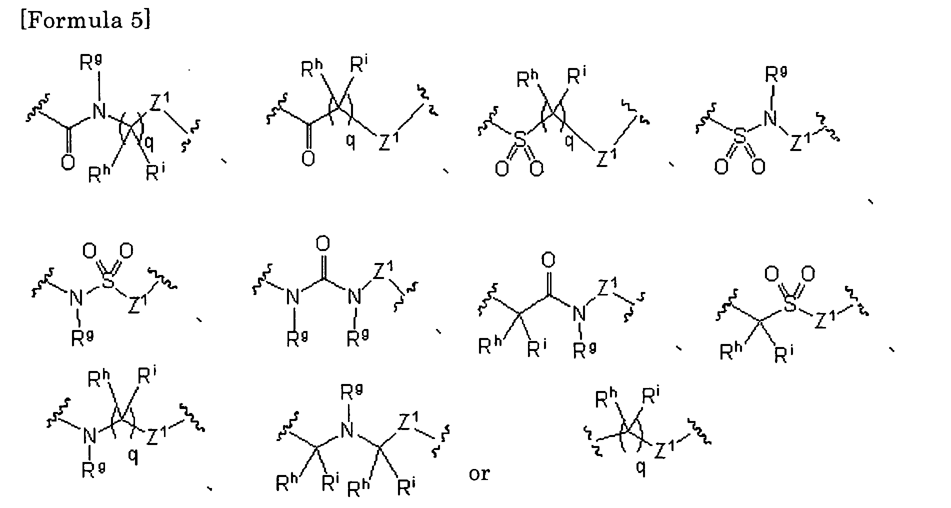 R^{g} are each independently hydrogen or optionally substituted lower alkyl,
   R^{h} and Rⁱ are each independently hydrogen or optionally substituted lower alkyl,
   q is an integer between 0 and 3,
   Z¹ is a bond, O, S or NRⁱ wherein Rⁱ is hydrogen, optionally substituted lower alkyl, optionally substituted acyl, optionally substituted lower alkylsulfonyl or optionally substituted arylsulfonyl,
   Ring E is optionally substituted aromatic carbocyclic diyl or optionally substituted aromatic heterocyclediyl,
   Y³ is a bond, optionally substituted lower alkylene which is optionally intervened by - O- or optionally substituted lower alkenylene,
   Z² is COOR^{c}, C(=NR^{c})NRⁿ OR^{o}, CONHCN or a group of the formula^{:} 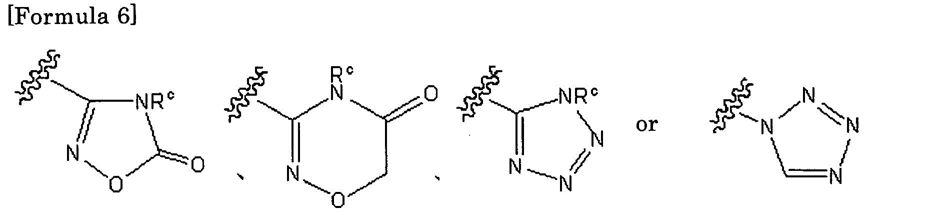 wherein R_{c}, Rₙ and Rₘ are each independently hydrogen, optionally substituted lower alkyl, optionally substituted lower alkenyl, optionally substituted aryl or optionally substituted heteroaryl,
   provided that
   a compound wherein a group of the formula: -Y² Z¹- is a group of the formula: 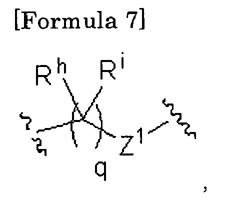 q is 0 and Z¹ is a bond is excluded.
(10) The pharmaceutical combination of any one of (1) to (7), wherein the compound having PPARδ agonistic activity is a compound of 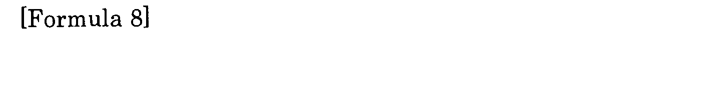 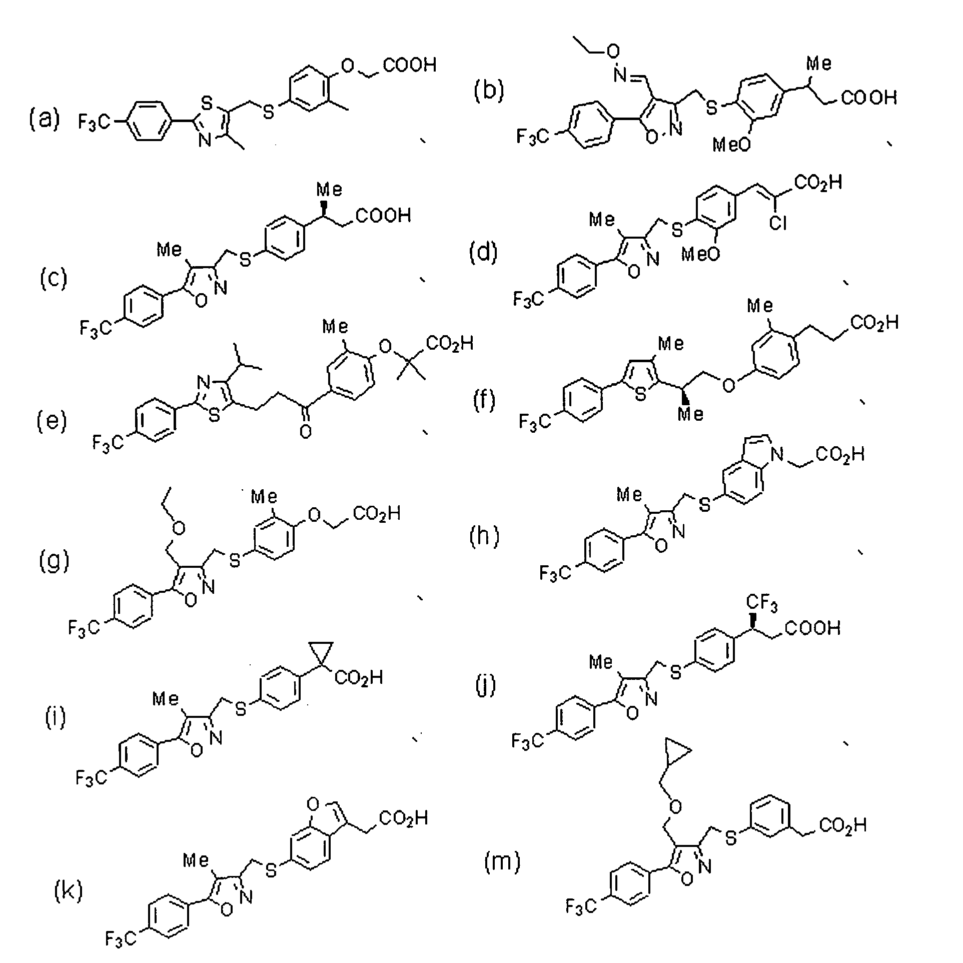 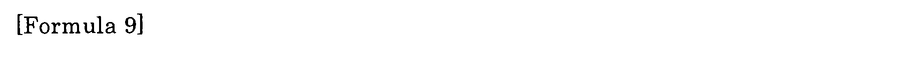 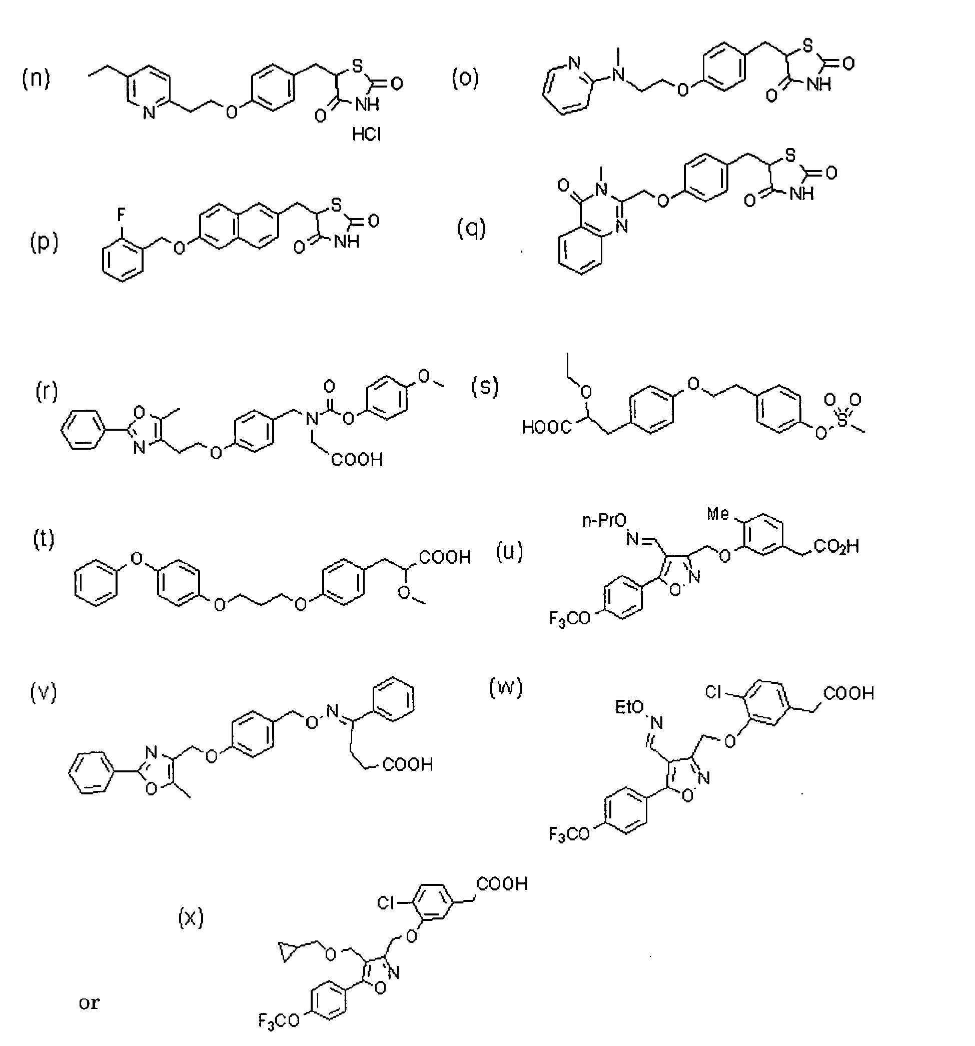 pharmaceutically acceptable salt or solvate thereof.
(11) A muscular disorder suppressant comprising vitamin K.

Furthermore, the present invention includes the following inventions.
(12) A suppressant of abnormal blood coagulation caused by a compound having PPARδ agonistic activity, comprising vitamin K.
(13) A method for suppressing abnormal blood coagulation caused by a compound having PPARδ agonistic activity, characterized by administering vitamin K.
(14) Use of vitamin K to suppress abnormal blood coagulation caused by a compound having PPARδ agonistic activity.
(15) A therapeutic and/or preventive agent for a disease relating to PPARδ, comprising the combination of a compound having PPARδ agonistic activity and vitamin K.
(16) A therapeutic and/or preventive method for a disease relating to PPARδ, characterized by administering a compound having PPARδ agonistic activity and vitamin K.
(17) Use of a compound having PPARδ agonistic activity and vitamin K to produce a therapeutic and/or preventive agent for a disease relating to PPARδ.
(18) A method for suppress muscular disorder, characterized by administering vitamin K.
(19) Use of vitamin K to suppress muscular disorder.
(20) A therapeutic and/or preventive agent for a disease relating to HMG-CoA reductase, comprising the combination of a statin compound and vitamin K.
(21) A therapeutic and/or preventive method for a disease relating to HMG-CoA reductase, characterized by administering a statin compound and vitamin K.
(22) Use of a statin compound and vitamin K to produce a therapeutic and/or preventive agent for a disease relating to HMG-CoA reductase.

A pharmaceutical combination of the present invention is useful as few side-effects, more appropriate, therapeutic and/or preventive agent for a disease which a compound having PPARδ agonistic activity can used as a medicinal preparation, for example, hyperlipidemia, diabetes, obesity, arteriosclerosis, atherosclerosis, hyperglycemia and/or syndrome X. Additionally, it is useful as a therapeutic and/or preventive agent for a disease causing muscular disorder. By combining with an agent causing muscular disorder (e.g., statin drugs or fibrate drugs), side-effects can be suppressed.

### Effect of the Invention

As the following test results show, pharmaceutical combinations of the present invention did not lead to abnormal blood coagulation caused by a compound having PPARδ agonistic activity. Furthermore, it suppressed muscular disorder. The pharmaceutical combinations of the present invention are very useful as a medicine which is few side-effects and more appropriate treatment and/or prevention especially for hyperlipidemia, diabetes, obesity, arteriosclerosis, atherosclerosis, hyperglycemia and/or syndrome X.

### Brief Description of the Drawings

[Figure 1] A vitamin K cycle and -glutamyl carboxylase.

### Best Mode for Carrying Out the Invention

"Vitamin K" means liposolubility vitamin having an activity to promote blood coagulation. It is vitamin which promotes prothrombin synthesis or production of blood coagulation factors in liver. Vitamin K deficiency causes impaired blood clotting. "Vitamin K" in the present invention is not especially restricted if it is vitamin classified as vitamin K. The examples are vitamin K1 (phylloquinone), vitamin K2 (menaquinone), vitamin K3 to K7 and derivatives thereof such as hydroquinone derivatives, esters, hydrates, alkylations, oxides and reduction compounds. Especially preferable examples are vitamin K1, K2 and K3.
Although vitamin K1 and K2 are obtained by isolating from natural products and vitamin K3 is obtained by synthesizing, they are commercially-supplied. It is known that vitamin K1 and K2 affect directly a vitamin K cycle, vitamin K3 is transformed to vitamin K2 by intestinal bacteria and then the vitamin K2 affect a vitamin K cycle.
Vitamin K comprising a pharmaceutical combination of the present invention has an activity to recover from blood coagulation caused by a compound having PPARkδ agonistic activity, especially vitamin K deficiency and hypoprothrombinaemia.

"A statin compound" is a general term of drugs to lower blood cholesterol by inhibiting HMG-CoA reductase. It is also called as a HMG-CoA reductase inhibitor. A statin compound inhibits HMG-CoA reductase, a rate-controlling enzyme in cholesterol biosynthesis which produces mevalonic acid. Therefore, it shows a strong cholesterol-lowering effect. Additionally, it is known that it lowers levels of neutral fat in serum at the same time. Examples are pravastatin, simvastatin, fulvastatin, atorvastatin, pitavastatin, lovastatin, cerivastatin, bervastatin, dalvastatin and mevastatin.

"A compound having PPARδ agonistic activity" means any compound which has PPARδ agonistic activity, pharmaceutically acceptable salt or solvate thereof. It is, for example, a compound whose EC₅₀ measured according to a method described in this description is 0.001 to 1000000 (nM) and preferably 0.1 to 100000 (nM). The following documents disclose thiophene, thiazole and isoxazole derivatives and related compounds thereof which have PPARδ agonistic activity and can be used with vitamin K as a pharmaceutical combination of the present invention, and their preparation methods. WO99/11255, WO01/00603, WO01/2518 WO02/080899, WO02/098840, WO02/102780, WO03/011807, WO03/016265, WO03/035603, WO03/074051, WO03/084916, WO03/099793, WO04/005253, WO04/022533, WO04/024939, WO04/037775, WO04/037776, WO04/056740, WO04/060871, WO04/063190, WO04/091604, WO05/016335, WO05/016881, WO02/059098, WO04/063166, WO02/014291, WO01/079197, WO03/033493, WO03/016291, WO04/063184, WO2005/054213, Japanese patent application number 2005-155739, PCT/JP2006/310198, Japanese patent application number 2005-246297, US4,687,777, US5,002,953, US5,594,016, WO97/41097 WO01/21602, WO99/62872, WO02/100813, WO2000-34266, US6,166,219, JP1997-295970, EP0745600, US5,886,014 and WO00/71540. They provide more information.

Example of "a compound having PPARδ agonistic activity" is a compound of the formula (I): 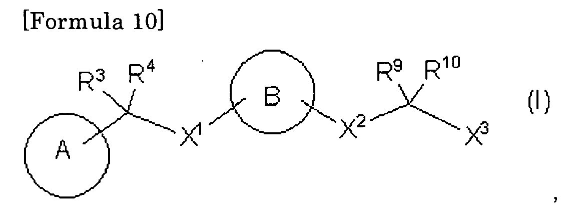 pharmaceutically acceptable salt or solvate thereof, wherein
Ring A is optionally substituted heteroaryl,
Ring B is optionally substituted aryl or optionally substituted heteroaryl,
R³ and R⁴ are each independently hydrogen, halogen, optionally substituted lower alkyl, optionally substituted lower alkenyl, optionally substituted lower alkynyl, optionally substituted aryl or optionally substituted heterocycle,
R⁹ and R¹⁰ are each independently hydrogen, halogen, cyano, optionally substituted lower alkyl, optionally substituted lower alkoxy, optionally substituted amino or optionally substituted aryl,
X¹ is -O-, -S-, -NR¹¹- wherein R¹¹ is hydrogen, optionally substituted lower alkyl, optionally substituted acyl, optionally substituted lower alkylsulfonyl or optionally substituted arylsulfonyl, -CR¹²R¹³CO-, -(CR¹²R¹³)mO-, -(CR¹² R¹³)mS-, or
-O(CR¹²R¹³)m- wherein R¹² and R¹³ are each independently hydrogen or optionally substituted lower alkyl and m is an integer between 1 and 3, -ON=CR¹⁴- wherein R¹⁴ is hydrogen or optionally substituted lower alkyl, or a group of the formula: 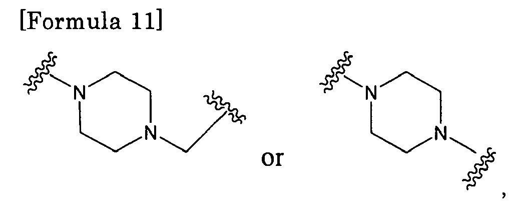 X² is a bond, -O-, -S-, -SO-, -SO₂ -, -CR²⁶ =CR²⁷ - wherein R²⁶ and R²⁷ are each independently hydrogen or optionally substituted lower alkyl, -NR¹⁴- wherein R¹⁴ is hydrogen, optionally substituted lower alkyl, optionally substituted acyl, optionally substituted lower alkylsulfonyl or optionally substituted arylsulfonyl, -CR¹⁵R¹⁶- wherein R¹⁵ and R¹⁶ are each independently hydrogen or optionally substituted lower alkyl or -COCR²⁴R²⁵- wherein R²⁴ and R²⁵ are each independently hydrogen or optionally substituted lower alkyl,
X³ is COOR¹⁷, C(=NR¹⁷)NR¹⁸0R¹⁹ or a group of the formula: 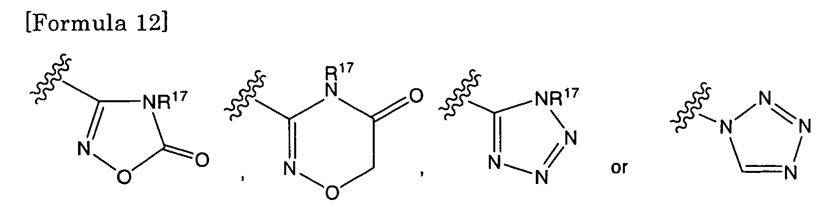 wherein R¹⁷ to R¹⁹ are each independently hydrogen or optionally substituted lower alkyl,
provided that
R⁹ and R¹⁶ can be joined together to form a bond,
R⁹ and R¹⁰ can be taken together to form a ring,
R⁹ and R²⁵ can be joined together to form a bond,
R⁹, R¹⁰ and R¹⁵ can be taken together with the neighboring carbon atom to form a ring,
R¹⁰ and R¹⁵ can be joined together to form a bond, and
R¹⁰ and R¹⁵ can be taken together with the neighboring carbon atom to form a ring.

Terms in the formula (I) are explained below.

The term "halogen" includes fluorine, chlorine, bromine and iodine. Fluorine or chlorine is especially preferable.

The term "lower alkyl" includes C1 to C10, preferably C1 to C6 and more preferably C1 to C3 straight or branched alkyl. Examples include methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl and n-decyl.

The term "lower alkenyl" includes C2 to C10, preferably C2 to C6 and more preferably C2 to C4 straight or branched alkenyl having one or more double bonds at arbitrary position(s). Examples include vinyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl and decenyl.

The term "lower alkynyl" includes C2 to C10, preferably C2 to C6 and more preferably C2 to C4 straight or branched alkynyl. Examples include ethynyl, propynyl, butynyl, pentynyl, hexynyl, heptynyl, octynyl, nonynyl and decenyl. These alkynyl have one or more triple bonds at arbitrary position(s) and can have double bond(s).

A substituent of "optionally substituted lower alkyl", "optionally substituted lower alkenyl" or "optionally substituted lower alkynyl" is halogen, hydroxy, optionally substituted lower alkoxy, optionally substituted lower alkynyloxy, amino, lower alkylamino, arylamino, heterocycle amino, acylamino, lower alkoxycarbonylamino, mercapto, lower alkylthio, acyl, acyloxy, optionally substituted imino, optionally substituted iminoxy, carboxy, lower alkoxycarbonyl, carbamoyl, lower alkylcarbamoyl, thiocarbamoyl, lower alkylthiocarbamoyl, carbamoyloxy, lower alkylcarbamoyloxy, thiocarbamoyloxy, lower alkylthiocarbamoyloxy, sulfamoyl, lower alkylsulfamoyl, lower alkylsulfonyl, lower alkylsulfonyloxy, cyano, nitro, optionally substituted cycloalkyl, cycloalkyloxy, optionally substituted aryl, optionally substituted aryloxy, optionally substituted arylthio, optionally substituted aryl lower alkoxy, optionally substituted arylsulfonyloxy or optionally substituted heterocycle
wherein the substituent is halogen, hydroxy, lower alkyl, halogeno lower alkyl, hydroxy lower alkyl, lower alkenyl, lower alkoxy, aryl lower alkoxy, halogeno lower alkoxy, carboxy, lower alkoxycarbonyl, carbamoyl, lower alkylcarbamoyl, arylcarbamoyl, acylamino, mercapto, lower alkylthio, amino, lower alkylamino, acyl, acyloxy, cyano, nitro, phenyl, heterocycle or the like. They can be substituted at arbitrary position(s) with one ore more substituent(s) selected from the above.

A substituent of "optionally substituted lower alkyl", "optionally substituted lower alkenyl", "optionally substituted lower alkynyl" or the like is preferably morpholino, piperidino, piperazino, furyl, thienyl or pyridyl.

A lower alkyl part of "halogeno lower alkyl", "hydroxy lower alkyl", "lower alkoxy", "lower alkynyloxy", "halogeno lower alkoxy", "aryl lower alkoxy", "hydroxy lower alkoxy", "lower alkylamino", "lower alkylthio", "lower alkylsulfonyl", "lower alkylsulfonyloxy", "lower alkylcarbamoyl", "lower alkylthiocarbamoyl", "lower alkylcarbamoyloxy", "lower alkylthiocarbamoyloxy", "lower alkylsulfamoyl", "lower alkoxycarbonyl" or "lower alkoxycarbonylamino" is same as the above "lower alkyl".

A substituent of "optionally substituted lower alkoxy", "optionally substituted lower alkoxycarbonyl", "optionally substituted lower alkylthio", "optionally substituted lower alkylsulfonyloxy", "optionally substituted imino" or "optionally substituted iminoxy" is same as a substituent of the above "optionally substituted lower alkyl".

The term "acyl" includes (a) C1 to C10, more preferably C1 to C6 and most preferably C1 to C3 straight or branched alkylcarbonyl or alkenylcarbonyl, (b) C4 to C9 and preferably C4 to C7 cycloalkylcarbonyl, (c) C7 to C11 arylcarbonyl and (d) formyl. Examples include formyl, acetyl, propionyl, butyryl, isobutyryl, valeryl, pivaloyl, hexanoyl, acryloyl, propioloyl, methacryloyl, crotonoyl, cyclopropylcarbonyl, cyclopropylcarbonyl, cyclooctylcarbonyl and benzoyl.

An acyl part of "acylamino" or "acyloxy" is same as the above "acyl".

A substituent of "optionally substituted acyl" is same as a substituent of the above "optionally substituted lower alkyl". Furthermore, cycloalkyl carbonyl and aryl carbonyl can be substituted with lower alkyl, halogeno lower alkyl, hydroxy lower alkyl, lower alkenyl, halogeno lower alkenyl and/or hydroxy lower alkenyl.

A substituent of "optionally substituted amino" is same as the above "optionally substituted lower alkyl". Furthermore, it can be lower alkyl, halogeno lower alkyl, hydroxy lower alkyl, lower alkenyl, halogeno lower alkenyl and/or hydroxy lower alkenyl.

A substituent of "optionally substituted carbamoyl", "optionally substituted thiocarbamoyl", "optionally substituted carbamoyloxy", "optionally substituted thiocarbamoyloxy" or "optionally substituted hydrazinocarbonyl" is same as the above "optionally substituted lower alkyl".

The term "cycloalkyl" includes C3 to C8 and preferably C5 or C6 cyclic alkyl. Examples are cyclopropyl, cyclobutyl, cyclopentyl, cyclohexyl, cycloheptyl and cyclooctyl.

The term "aryl" includes phenyl, naphthyl, anthryl and phenanthryl. Additionally, it includes aryl condensed with the other non-aromatic hydrocarbon ring. Examples are indanyl, indenyl, biphenylyl, acenaphthenyl and fluorenyl. In case that aryl is condensed with the other non-aromatic hydrocarbon ring, bonds can be attached to any of the rings. The preferable example of aryl is phenyl.

The term "heteroaryl" includes 5- or 6-membered aromatic ring having 1 to 4 heteroatom(s) selected from the group consisting of N, O and S (e.g., furan, thiophene, pyrrole, oxazole, thiazole, imidazole, pyrazole, triazol, pyridine, pyridazine, pyrimidine, pyrazine or triazine). In case that heteroaryl is condensed with other nonaromatic hydrocarbon ring, the bonds can be attached to any of the rings. Preferable examples are thiophene, thiazole and isoxazole.

A substituent of "optionally substituted cycloalkyl", "optionally substituted aryl" or "optionally substituted heteroaryl" is the same as a substituent of the above "optionally substituted lower alkyl" as long as there is not a special provision. Furthermore, it can be lower alkyl, halogeno lower alkyl, hydroxy lower alkyl, lower alkenyl, halogeno lower alkenyl, hydroxy lower alkenyl, alkylenedioxy and/or oxo.

An aryl part of "aryloxy", "arylthio", "aryl lower alkoxy", "arylamino" or "arylsulfonyloxy" is the same as aryl part of the above "aryl".

A substituent of "optionally substituted aryloxy", "optionally substituted arylthio" or "optionally substituted arylsulfonyloxy" is the same as the substituent of the above "optionally substituted aryl" as long as there is not a special provision.

The term "heterocycle" includes heterocycle having 1 or more hetero atom(s) selected from O, S and N in the ring, for example, 5- or 6-membered heteroaryl such as pyrrolyl, imidazolyl, pyrazolyl, pyridyl, pyridazinyl, pyrimidinyl, pyradinyl, triazolyl, triazinyl, tetrazolyl, isoxazolyl, oxazolyl, oxadiazolyl, isothiazolyl, thiazolyl, thiadiazolyl, furyl and thienyl; bicyclic condensed heterocycle such as indolyl, isoindolyl, indazolyl, indolizinyl, quinolyl, isoquinolyl, cinnolinyl, phthalazinyl, quinazolinyl, naphthyridinyl, quinoxalinyl, prinyl, pteridinyl, benzopyranyl, benzimidazolyl, benzisoxazolyl, benzoxazolyl, benzoxadiazolyl, benzoisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyradino pyridazinyl, quinazolinyl, tetrahydroquinolyl and tetrahydrobenzothienyl; tricyclic condensed heterocycle such as carbazolyl, acridinyl, xanthenyl, phenothiazinyl, phenoxathiinyl, phenoxazinyl and dibenzofuryl; and non-aromatic heterocycle such as indolinyl, dioxanyl, thiiranyl, oxyranyl, oxathiolanyl, azetidinyl, thianyl, pyrrolidinyl, pyrrolinyl, imidazolidinyl, imidazolinyl, pyrazolidinyl, pyrazolinyl, piperidyl, piperidino, piperazinyl, piperadino, morpholinyl, morpholino, oxadiadinyl and dihydropyridyl. In case that heterocycle is a condensed ring, the bonds can be attached to any of the rings.

Preferable examples of "heterocycle" for R¹ and R² are pyridyl, morpholino, piperazino and piperidino.

A substituent of "optionally substituted heterocycle" is the same as the substituent of the above "optionally substituted aryl".

A heterocycle part of "heterocycleamino" is the same as the above "heterocycle".

"Ring A" is optionally substituted heteroaryl. The preferable examples are optionally substituted thiophene, oxazole, isoxazole, thiazole and imidazole.
Examples of "a substituent" of Ring A are hydrogen, halogen, hydroxy, cyano, optionally substituted lower alkyl, optionally substituted lower alkenyl, optionally substituted lower alkynyl, optionally substituted lower alkoxy, carboxy, optionally substituted lower alkoxycarbonyl, optionally substituted lower alkylthio, optionally substituted acyl, optionally substituted amino, optionally substituted carbamoyl, optionally substituted thiocarbamoyl, optionally substituted carbamoyloxy, optionally substituted thiocarbamoyloxy, optionally substituted hydrazinocarbonyl, optionally substituted lower alkylsulfonyloxy, optionally substituted arylsulfonyloxy, optionally substituted aryl, optionally substituted aryloxy, optionally substituted arylthio and optionally substituted heterocycle.

"Ring B" is optionally substituted aryl or optionally substituted heteroaryl. The preferable examples are optionally substituted phenyl, pyrimidine and thiophene wherein the substituent is hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle or the like, and the following fused rings.

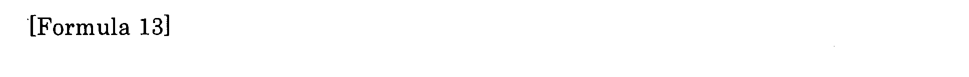

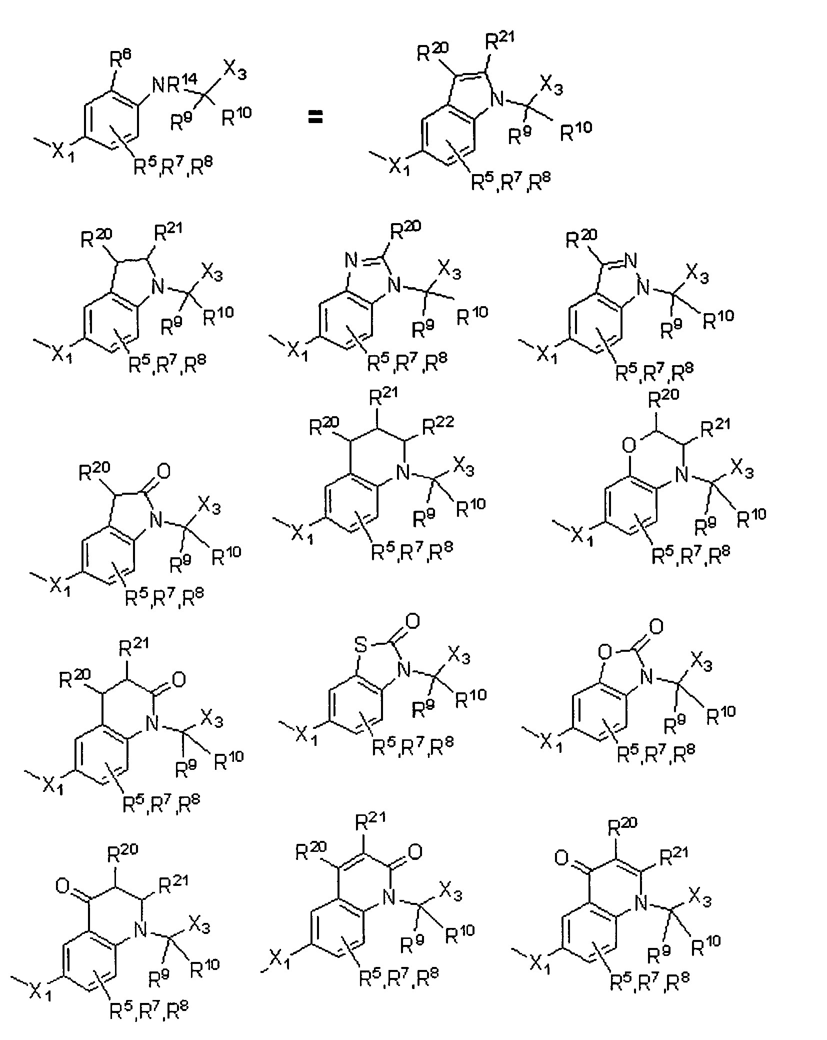

In the above formula,
R⁵, R⁷ and R⁸ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle, R⁹ and R¹⁰ are each independently hydrogen, halogen, cyano, optionally substituted lower alkyl, optionally substituted lower alkoxy, optionally substituted amino or optionally substituted aryl,
R²⁰ to R²² are each independently hydrogen, halogen, hydroxy, cyano, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted imino, optionally substituted aryl, optionally substituted aryloxy, optionally substituted arylthio or optionally substituted heterocycle,
X¹ is -O-, -S-, -NR¹¹- wherein R¹¹ is hydrogen, optionally substituted lower alkyl, optionally substituted acyl, optionally substituted lower alkylsulfonyl or optionally substituted arylsulfonyl , -CR¹²R¹³CO-, -(CR¹²R¹³)mO-, -(CR¹²R¹³)mS- or -O(CR¹²R¹³)m-
wherein R¹² and R¹³ are each independently hydrogen or optionally substituted lower alkyl, and m is an integer between 1 and 3 (-O- or -S- is preferable and -S- is especially preferable), and X³ is COOR¹⁷ wherein R¹⁷ is hydrogen or optionally substituted lower alkyl.

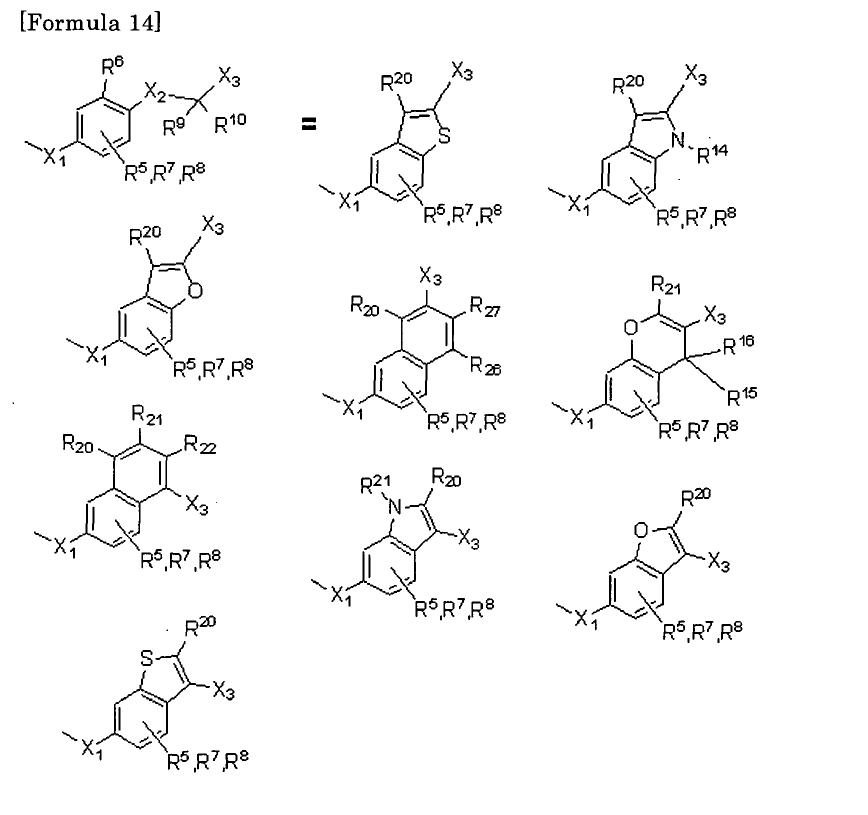

In the above formula,
R⁵, R⁷, R⁸and R²⁰ to R²² are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
X¹ is -O-, -S-, -NR¹¹- wherein R¹¹ is hydrogen, optionally substituted lower alkyl, optionally substituted acyl, optionally substituted lower alkylsulfonyl or optionally substituted arylsulfonyl, -CR¹²R¹³CO-, -(CR¹²R¹³)mO-, -(CR¹²R¹³)mS- or -O(CR¹²R¹³)m-
wherein R¹² and R¹³ are each independently hydrogen or optionally substituted lower alkyl, and m is an integer between 1 and 3 (-O- or -S- is preferable and -S- is especially preferable),
R¹⁴ is hydrogen, optionally substituted lower alkyl, optionally substituted acyl, optionally substituted lower alkylsulfonyl or optionally substituted arylsulfonyl,
R¹⁵, R¹⁶, R²⁶ and R²⁷ are each independently hydrogen or optionally substituted lower alkyl, and
X³ is COOR¹⁷ wherein R¹⁷ is hydrogen or optionally substituted lower alkyl.

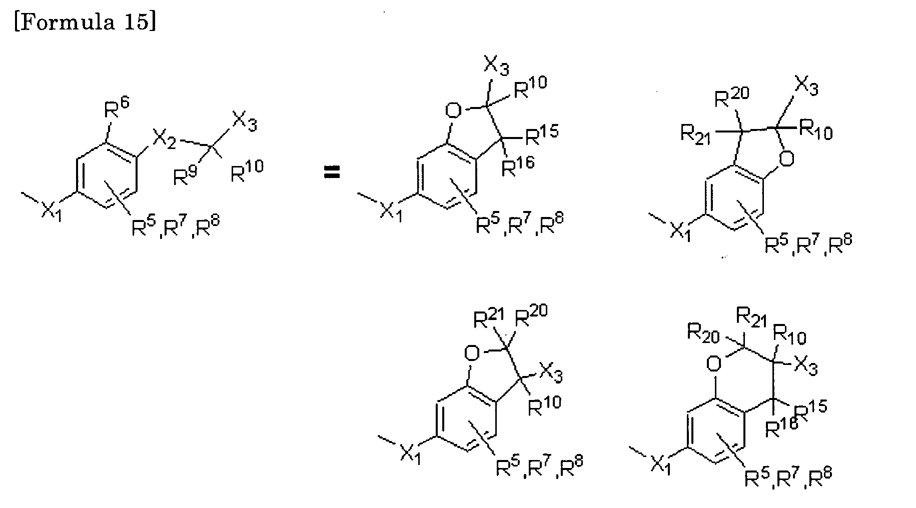

In the above formula,
R⁵, R⁷, R⁸, R²⁰ and R²¹ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
R¹⁰ is hydrogen, halogen, cyano, optionally substituted lower alkyl, optionally substituted lower alkoxy, optionally substituted amino or optionally substituted aryl, X¹ is -O-, -S-, -NR¹¹- wherein R¹¹ is hydrogen, optionally substituted lower alkyl, optionally substituted acyl, optionally substituted lower alkylsulfonyl or optionally substituted arylsulfonyl, -CR¹²R¹³CO-, -(CR¹²R¹³)mO-, -(CR¹²R¹³)mS- or -O(CR¹²R¹³)ₘ- wherein R¹² and R¹³ are each independently hydrogen or optionally substituted lower alkyl, and m is an integer between 1 and 3 (-O- or -S- is preferable and -S- is especially preferable),
R¹⁵ and R¹⁶ are each independently hydrogen or optionally substituted lower alkyl, and
X³ is COOR¹⁷ wherein R¹⁷ is hydrogen or optionally substituted lower alkyl.

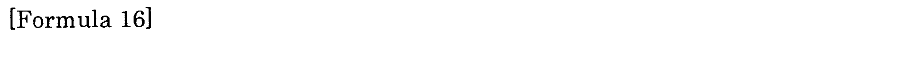

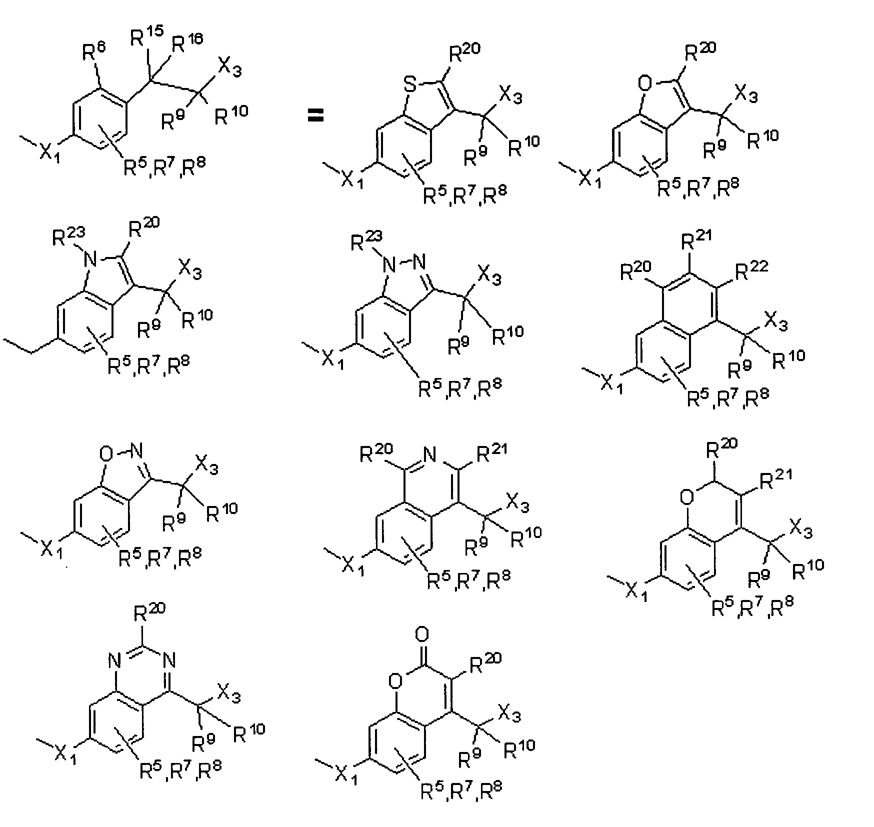

In the above formula,
R⁵, R⁷, R⁸ and R²⁰ to R²² are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
R⁹ and R¹⁰ are each independently hydrogen, halogen, cyano, optionally substituted lower alkyl, optionally substituted lower alkoxy, optionally substituted amino or optionally substituted aryl,
R²³ is hydrogen, optionally substituted lower alkyl, optionally substituted lower alkenyl, optionally substituted lower alkynyl, optionally substituted acyl, optionally substituted lower alkylsulfonyl or optionally substituted arylsulfonyl, optionally substituted amino, optionally substituted aryl or optionally substituted heterocycle, X¹ is -O-, -S-, -NR¹¹- wherein R¹¹ is hydrogen, optionally substituted lower alkyl, optionally substituted acyl, optionally substituted lower alkylsulfonyl or optionally substituted arylsulfonyl, -CR¹²R¹³CO⁻, -(CR¹²R¹³)mO-, -(CR¹²R¹³)mS- or -O(CR¹²R¹³)m-
wherein R¹² and R¹³ are each independently hydrogen or optionally substituted lower alkyl, and m is an integer between 1 and 3 (-O- or -S- is preferable and -S- is especially preferable), and
X³ is COOR¹⁷ wherein R¹⁷ is hydrogen or optionally substituted lower alkyl.

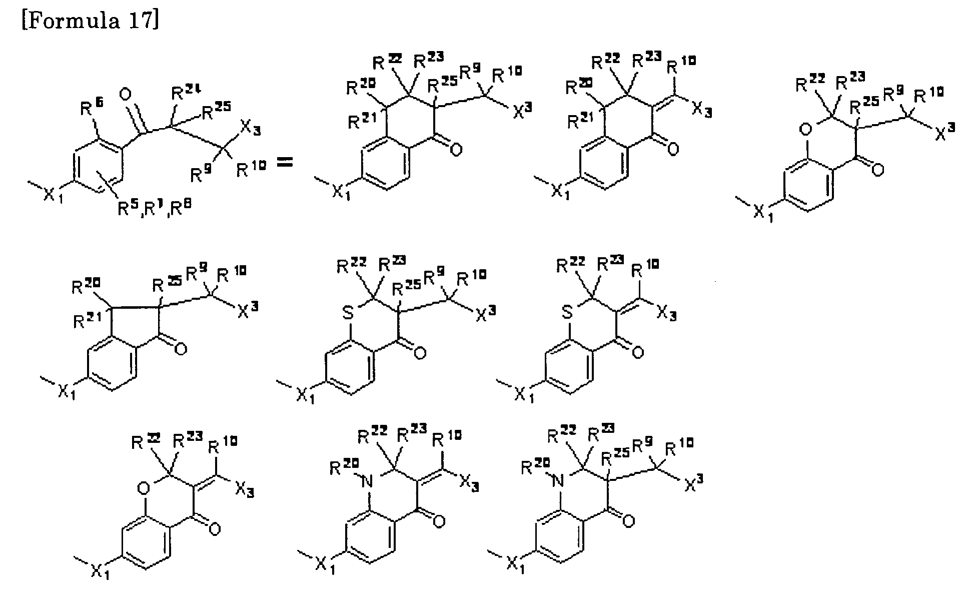

In the above formula,
R5, R⁷, R⁸ and R²⁰ to R²³ are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
R⁹, R¹⁰ and R²⁵ are each independently hydrogen, halogen, cyano, optionally substituted lower alkyl, optionally substituted lower alkoxy, optionally substituted amino or optionally substituted aryl,
X¹ is -O-, -S-, -NR¹¹- wherein R¹¹ is hydrogen, optionally substituted lower alkyl, optionally substituted acyl, optionally substituted lower alkylsulfonyl or optionally substituted arylsulfonyl, -CR¹²R¹³CO-, -(CR¹²R¹³)mO-, -(CR¹²R¹³)mS- or -O(CR¹²R¹³)m-
wherein R¹² and R¹³ are each independently hydrogen or optionally substituted lower alkyl, and m is an integer between 1 and 3 (-O- or -S- is preferable and -S- is especially preferable), and
X³ is COOR¹⁷ wherein R¹⁷ is hydrogen or optionally substituted lower alkyl.

"R⁹ and R²⁵ can be joined together to form a bond" or "R²⁵ and R⁹ can be joined together to form a bond" means 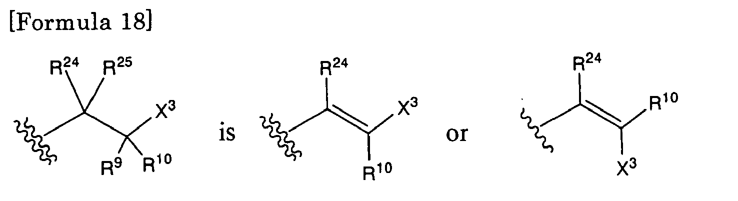 wherein
R¹⁰ and R²⁴ are each independently hydrogen, halogen, cyano, optionally substituted lower alkyl, optionally substituted lower alkoxy, optionally substituted amino or optionally substituted aryl, and
X³ is COOR¹⁷ wherein R¹⁷ is hydrogen or optionally substituted lower alkyl.

"R⁹ and R¹⁰ can be taken together to form a ring" means that R⁹ and R¹⁰ form a 3- to 7-membered ring with 0 to 3 hetero atom(s). The preferable examples of the ring are optionally substituted C3 to C7 carbon monocycle and optionally substituted heteromonocycle. They include cycloalkane (cyclopropane, cyclobutane, cyclopentane, cyclohexane and cycloheptane) and oxane. A substituent of "optionally substituted C3 to C7 carbon monocycle (especially optionally substituted 3-membered ring)" or "optionally substituted heteromonocycle" is the same as the substituent on benzene ring of the formula (I). Examples of a substituent are halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio, optionally substituted heterocycle and oxo. Halogen, hydroxy, optionally substituted lower alkoxy, optionally substituted lower alkylthio or optionally substituted lower alkyl is especially preferable.
The preferable examples of "optionally substituted C3 to C7 carbon monocycle (especially optionally substituted 3-membered ring)" and "optionally substituted heteromonocycle" are 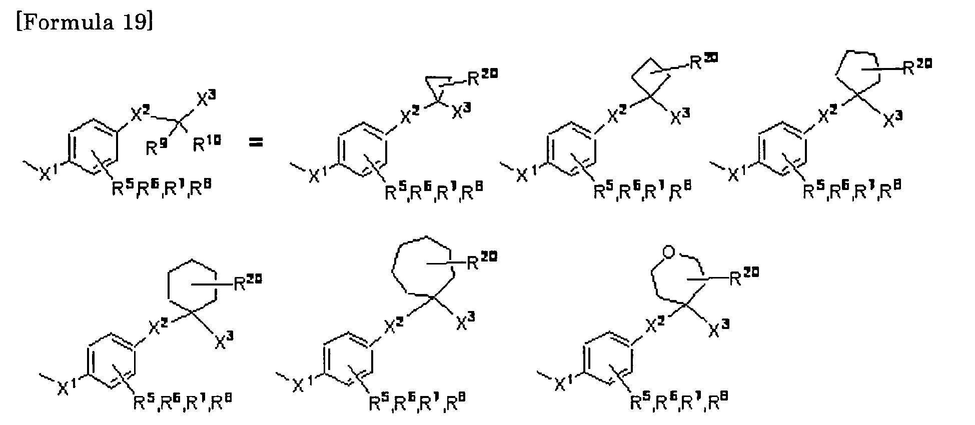 wherein
R⁵, R⁶, R⁷, R⁸ and R²⁰ are each independently hydrogen, halogen, hydroxy, optionally substituted lower alkyl, optionally substituted lower alkenyl, optionally substituted lower alkynyl, optionally substituted lower alkoxy, optionally substituted lower
alkylthio, optionally substituted acyl, optionally substituted amino, optionally substituted aryl, optionally substituted aryloxy, optionally substituted arylthio or optionally substituted heterocycle,
X¹ is -O-, -S-, -NR¹¹- wherein R¹¹ is hydrogen, optionally substituted lower alkyl, optionally substituted acyl, optionally substituted lower alkylsulfonyl or optionally substituted arylsulfonyl, -CR¹²R¹³CO-, -(CR¹²R¹³)mO⁻, -(CR¹²R¹³)mS- or -O(CR¹²R¹³)m-wherein R¹² and R¹³ are each independently hydrogen or optionally substituted lower alkyl, and m is an integer between 1 and 3 (-O- or -S- is preferable and -S- is especially preferable),
X² is a bond, -O-, -S-, -SO-, -SO₂- -C=C-, -NR¹⁴- wherein R¹⁴ is hydrogen, optionally substituted lower alkyl, optionally substituted acyl, optionally substituted lower alkylsulfonyl or optionally substituted arylsulfonyl , -CR¹⁵R¹⁶- wherein R¹⁵ and R¹⁶ are each independently hydrogen or optionally substituted lower alkyl or -COCR²³R²⁴-
wherein R²³ and R²⁴ are each independently hydrogen or optionally substituted lower alkyl and
X³ is COOR¹⁷ wherein R¹⁷ is hydrogen or optionally substituted lower alkyl.

"R¹⁰ and R¹⁵ can be taken together with the neighboring carbon atom to form a ring" or "R¹⁵ and R¹⁰ can be taken together with the neighboring carbon atom to form a ring" means that R¹⁵ and R¹⁰ form a 4- to 7-membered ring having 0 to 3 heteroatom(s). The preferable example of the ring is optionally substituted C3 to C7 carbon monocycle or optionally substituted heteromonocycle. Examples are thiophene, pyrimidine, furan, pyridine, imidazole, isothiazole, isoxazole, pyridazine, pyrazine, thiazole and oxazole.
The case that R¹⁶ and R⁹ are joined together to form a bond or the case that R⁹, R¹⁰ and R¹⁵ can be taken together with the neighboring carbon atom to form a ring is especially preferable. The substituent of "optionally substituted C3 to C7 carbon monocycle" or "optionally substituted heteromonocycle" is same as a substituent on benzene ring of the formula (I). Examples of the substituent are halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio, optionally substituted heterocycle and oxo. Halogen, hydroxy, optionally substituted lower alkoxy, optionally substituted lower alkylthio or optionally substituted lower alkyl is especially preferable.
The preferable examples of "optionally substituted C3 to C7 carbon monocycle (especially optionally substituted phenyl)" or "optionally substituted heteromonocycle" are 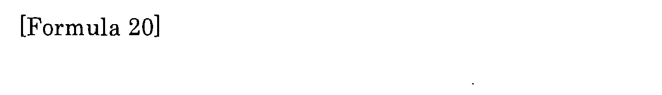 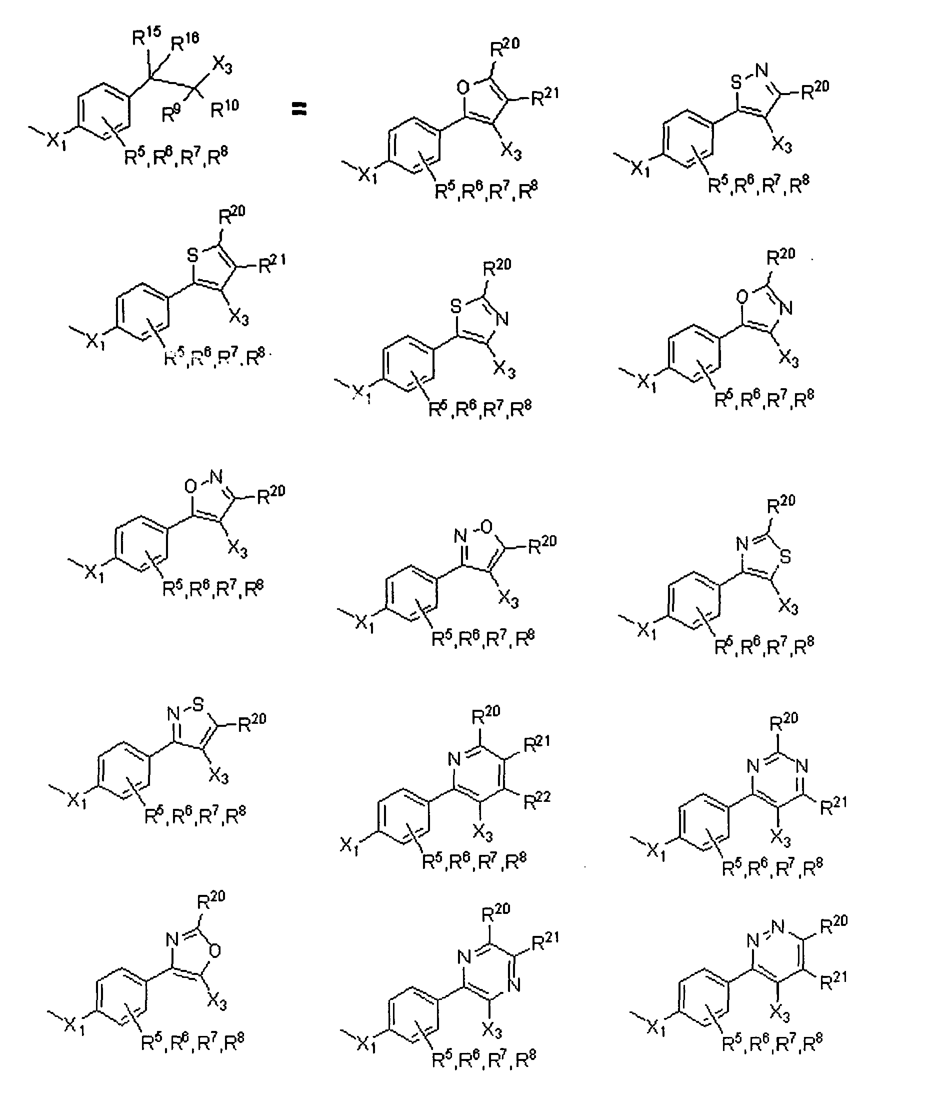 wherein
R⁵, R⁶, R⁷, R⁸ and R²⁰ to R²² are each independently hydrogen, halogen, hydroxy, optionally substituted lower alkyl, optionally substituted lower alkenyl, optionally substituted lower alkynyl, optionally substituted lower alkoxy, optionally substituted lower alkylthio, optionally substituted acyl, optionally substituted amino, optionally substituted aryl, optionally substituted aryloxy, optionally substituted arylthio or optionally substituted heterocycle,
X¹ is -O-, -S-, -NR¹¹- wherein R¹¹ is hydrogen, optionally substituted lower alkyl, optionally substituted acyl, optionally substituted lower alkylsulfonyl or optionally substituted arylsulfonyl, -CR¹²R¹³CO-, -(CR¹²R¹³)mO-, -(CR¹²-R¹³)mS- or -O(CR¹²R¹³)m-
wherein R¹² and R¹³ are each independently hydrogen or optionally substituted lower alkyl and m is an integer between 1 and 3 (-O- or -S- is preferable and -S- is especially preferable), and
X³ is COOR¹⁷ wherein R¹⁷ is hydrogen or optionally substituted lower alkyl.

"R⁹ and R¹⁶ can be joined together to form a bond" or "R¹⁶ and R⁹ can be joined together to form a bond" means 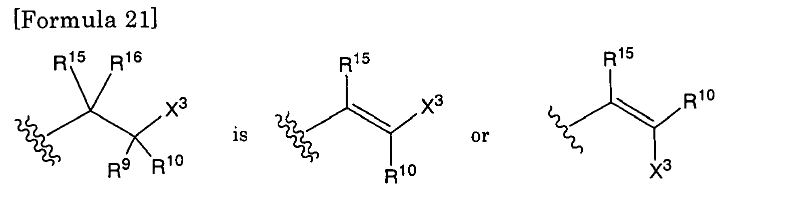 wherein
R¹⁰ and R¹⁵ are each independently hydrogen, halogen, cyano, optionally substituted lower alkyl, optionally substituted lower alkoxy, optionally substituted amino or optionally substituted aryl, and
X³ is COOR¹⁷ wherein R¹⁷ is hydrogen or optionally substituted lower alkyl.

"R¹⁶ and R⁹ are taken together to form a bond and R¹⁵ and R¹⁰ are taken together to form a bond" means that 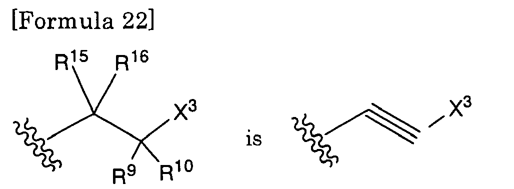 wherein X³ is COOR¹⁷ wherein R¹⁷ is hydrogen or optionally substituted lower alkyl.

Other example of "a compound having PPARδ agonistic activity" is a compound of the formula (II): 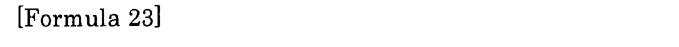 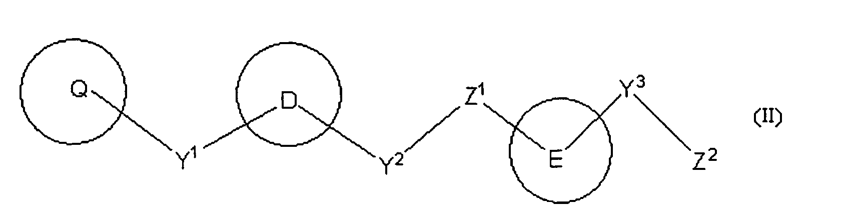 pharmaceutically acceptable salt or solvate thereof, wherein
Ring Q is monocyclic aryl substituted with at least one of R^{b} and optionally substituted with other group(s), monocyclic heteroaryl substituted with at least one of R^{b} and optionally substituted with other group(s) wherein each R^{b} is optionally substituted aryl, optionally substituted aralkyl, optionally substituted aryloxy, optionally substituted arylthio, optionally substituted heteroaryl, optionally substituted heteroaralkyl, optionally substituted heteroaryloxy or optionally substituted heteroarylthio, substituted fused aryl or substituted fused heteroaryl,
Y¹ is a bond or NR^{f} - wherein R^{f} is hydrogen or optionally substituted lower alkyl, Ring D is optionally substituted nonaromatic heterocyclediyl, provided that Ring Q binds with a nitrogen atom of Ring D when Y¹ is a bond,
a group of the formula: -Y² Z¹- is a group of 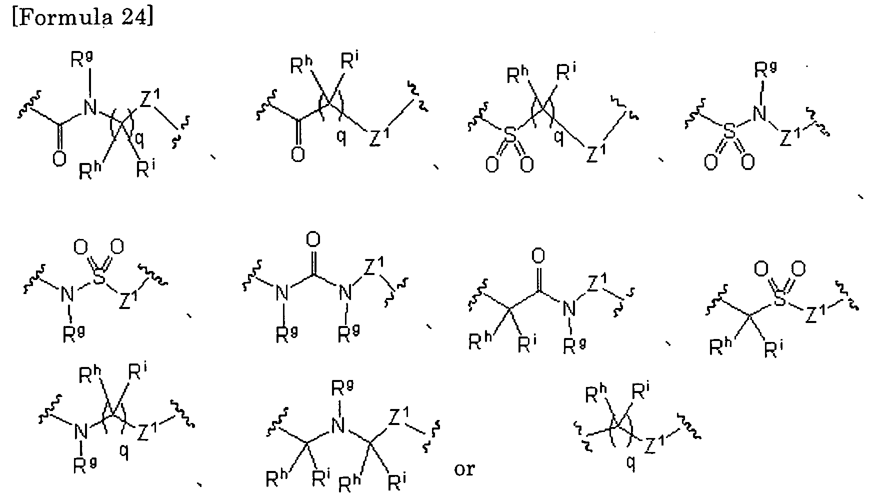 R^{g} is each independently hydrogen or optionally substituted lower alkyl,
R^{h} and Rⁱ are each independently hydrogen or optionally substituted lower alkyl,
q is an integer between 0 and 3,
Z¹ is a bond, O, S or NRⁱ wherein Rⁱ is hydrogen, optionally substituted lower alkyl, optionally substituted acyl, optionally substituted lower alkylsulfonyl or optionally substituted arylsulfonyl,
Ring E is optionally substituted aromatic carbocyclic diyl or optionally substituted aromatic heterocyclediyl,
Y³ is a bond, optionally substituted lower alkylene which is optionally intervened by -O- or optionally substituted lower alkenylene,
Z² is COOR^{c}, C(=NR^{c})NRⁿ OR^{o}, CONHCN or a group of the formula: 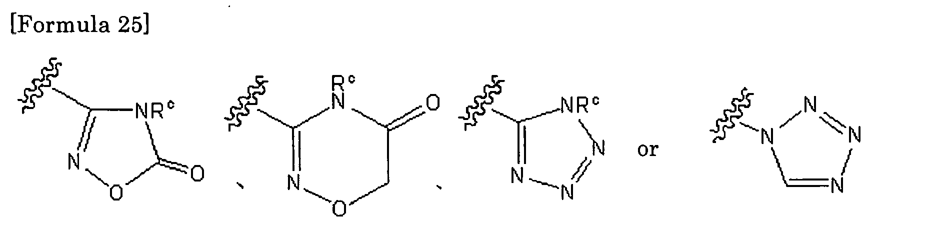 wherein R^{c}, Rⁿ and R^{m} are each independently hydrogen, optionally substituted lower alkyl, optionally substituted lower alkenyl, optionally substituted aryl or optionally substituted heteroaryl,
provided that
a compound wherein a group of the formula: -Y² Z¹- is a group of the formula: 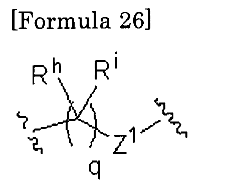 q is 0 and Z¹ is a bond is excluded.

Terms in The formula (II) are explained below.

The term "monocyclic aryl" means C6 to C12 monocyclic aromatic carbon ring.

### Example is phenyl or the like.

The term "fused aryl" means aromatic carbon ring which 1 to 4 monocyclic aromatic carbon ring (C6 to C12 monocyclic aromatic carbon ring) is condensed with C6 to C12 monocyclic aromatic carbon ring. Examples are naphthyl, anthryl and phenanthryl. The bonds can be attached to any of the rings. Naphthyl is preferable.
The term "aryl" means the above "monocyclic aryl" and the above "fused aryl".
The term "aralkyl" means the above "alkyl" substituted with 1 to 3 of the above "aryl". Examples are benzyl, phenethyl, phenylpropyl and trityl.

The term "monocyclic heteroaryl" means 4- to 8-membered monocyclic aromatic heterocycle having 1 or more hetero atom(s) selected from O, S and N in the ring. Examples are pyrrolyl, imidazolyl, pyrazolyl, pyridyl, pyridazinyl, pyrimidinyl, pyrazinyl, triazolyl, triazinyl, tetrazolyl, isoxazolyl, oxazolyl, oxadiazolyl, isothiazolyl, thiazolyl, thiadiazolyl, furyl and thienyl. 5- or 6-membered monocyclic aromatic heterocycle is especially preferable.
The term "fused heteroaryl" means a group derived from condensed aromatic heterocycle which aromatic carbon ring (aromatic carbon ring derived from the above "aryl") or aromatic heterocycle (4- to 8-membered aromatic heterocycle having 1 or more hetero atom(s) selected from O, S and N in the ring) is condensed with monocyclic aromatic heterocycle derived from the above "monocyclic heteroaryl". Examples are indolyl, isoindolyl, indazolyl, indolizinyl, quinolyl, isoquinolyl, cinnolinyl, phthalazinyl, quinazolinyl, naphthyridinyl, quinoxalinyl, prinyl, pteridinyl, benzopyranyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyradino pyridazinyl, quinazolinyl, tetrahydroquinolyl, tetrahydrobenzothienyl, carbazolyl, acridinyl, xanthenyl, phenothiazinyl, phenoxathiinyl, phenoxazinyl and dibenzofuryl. In case of "fused heteroaryl", the bonds can be attached to any of the rings. A condensed fused heteroaryl which benzene ring is condensed with 5- or 6-membered monocyclic aromatic heterocycle is especially preferable.
The term "heteroaryl" means the above "monocyclic heteroaryl" and "fused heteroaryl".
The term "heteroaralkyl" means the above "alkyl" substituted with 1 to 3 of the above "heteroaryl".
The term "nonaromatic heterocycle" means a condensed nonaromatic heterocycle which aromatic carbon ring (aromatic carbon ring derived from the above "aryl"), aromatic heterocycle (4- to 8-membered aromatic heterocycle having 1 or more hetero atom(s) selected from O, S and N in the ring), monocyclic nonaromatic heterocycle (monocyclic nonaromatic heterocycle derived from the above "monocyclic nonaromatic heterocycle") or cycloalkane (a ring derived from the below "cycloalkyl") is condensed with 4- to 8-membered monocyclic nonaromatic heterocycle having 1 or more hetero atom(s) selected from O, S and N in the ring or the above "monocyclic nonaromatic heterocycle". Examples are indolinyl, dioxanyl, thiiranyl, oxyranyl, oxathiolanyl, azetidinyl, thianyl, pyrrolidinyl, pyrrolinyl, imidazolidinyl, imidazolinyl, pyrazolidinyl, pyrazolinyl, piperidyl, piperidino, piperazinyl, piperadino, morpholinyl, morpholino, oxadiadinyl and dihydropyridyl.
The term "heterocycle" include the above "heteroaryl" and the above "nonaromatic heterocycle". Examples are morpholino, piperidino, piperadino, furyl, thienyl and pyridyl.

The term "nonaromatic heterocyclediyl" includes a bivalent group derived by removing 2 hydrogen atoms from 4- to 10-membered nonaromatic heterocycle having 1 or more hetero atom(s) selected from O, S and N in the ring. The nonaromatic heterocycle can be bridged by alkylene. The preferable examples are piperidinediyl, piperadinediyl, morpholinediyl, dioxanediyl, pyrrolidinediyl, pyrrolinediyl, imidazolinediyl and imidazolidinediyl. Examples of "nonaromatic heterocyclediyl" of Ring D are the following groups. 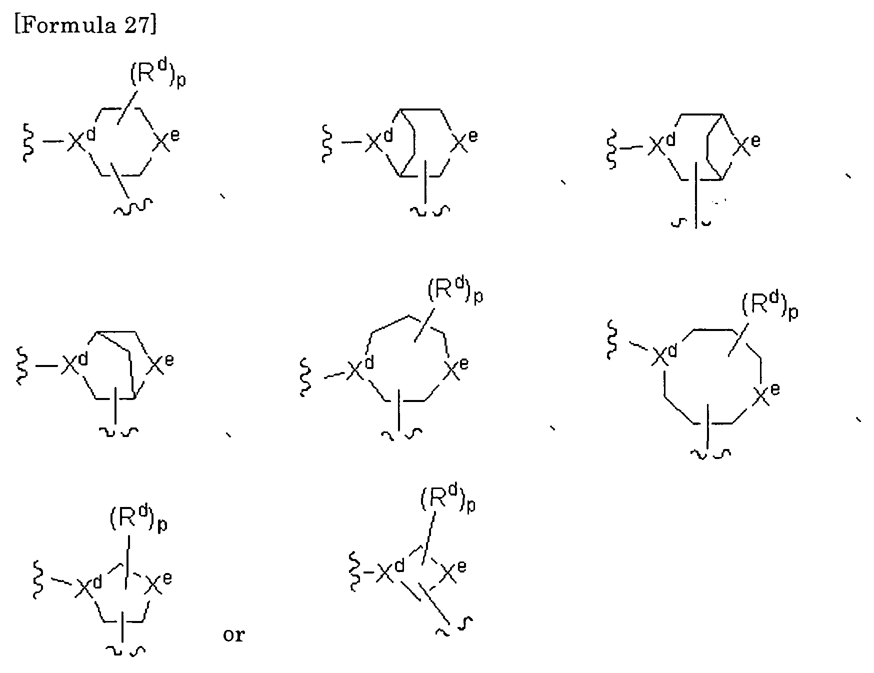 wherein
X^{d} is N or CR^{e} wherein R^{e} is hydrogen or optionally substituted lower alkyl,
X^{e} is O, S, NR^{p} or CR^{q} R^{r} wherein R^{p} to R^{r} are each independently hydrogen or optionally substituted lower alkyl,
provided that
a group wherein X^{d} is CR^{e} and X^{e} is CR^{q} R^{r} is excluded.
R^{d} are each independently, halogen, optionally substituted lower alkyl, optionally substituted lower alkenyl, optionally substituted lower alkynyl, optionally substituted lower alkoxy or optionally substituted aryl, and
p is an integer between 0 and 2.
In the above, the bond from X^{d} binds with Y¹ and the other bond binds with Y². When X^{e} is NR^{p} or CR^{q} R^{r}, the other bond can bind with X^{e}. The other bond preferably binds with X^{e}.

The term "aromatic carbocyclic diyl" includes a bivalent group derived by removing a hydrogen atom from the above "aryl". Examples are phenylene and naphthylene. Phenylene is preferable.

The term "aromatic heterocyclediyl" includes a bivalent group derived by removing a hydrogen atom from the above "heteroaryl". Examples are pyrroldiyl, imidazolediyl, pyrazolediyl, pyridinediyl, pyridazinediyl, pyrimidinediyl, pyrazinediyl, triazolediyl, triazinediyl, isoxazolediyl, oxazolediyl, oxadiazolediyl, isothiazolediyl, thiazolediyl, thiadiazolediyl, furandiyl, thiophenediyl, indolediyl, benzofurandiyl and benzothiophenediyl. Preferred is indolediyl, benzofurandiyl, benzothiophenediyl, furandiyl or thiophenediyl. Especially preferred is monocyclic aromatic heterocyclediyl. More preferred is furandiyl (especially furan-2,5-diyl) or thiophenediyl (especially thiophene-2,5-diyl).

The term "lower alkyl" includes C1 to C10, preferably C1 to C6 and more preferably C1 to C3 straight or branched alkyl. Examples include methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl and n-decyl.

The term "lower alkenyl" includes C2 to C10, preferably C2 to C6 and more preferably C2 to C4 straight or branched alkenyl having one or more double bonds at arbitrary positions. Examples include vinyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl and decenyl.

The term "lower alkynyl" includes C2 to C10, preferably C2 to C6 and more preferably C2 to C4 straight or branched alkynyl. Examples include ethynyl, propynyl, butynyl, pentynyl, hexynyl, heptynyl, octynyl, nonynyl and decenyl. These alkynyl have one or more triple bonds at arbitrary positions and can have double bonds.

The term "cycloalkyl" includes C3 to C9 and preferably C3 or C6 cyclic alkyl. Examples are cyclopropyl, cyclobutyl, cyclopentyl, cyclohexyl, cycloheptyl and cyclooctyl.

The term "acyl" includes (a) carbonyl substituted with the above "alkyl" or "alkenyl", (b) carbonyl substituted with the above "cycloalkyl", (c) carbonyl substituted with the above "aryl" and (d) formyl. Examples include formyl, acetyl, propionyl, butyryl, isobutyryl, valeryl, pivaloyl, hexanoyl, acryloyl, propioloyl, methacryloyl, crotonoyl, cyclopropylcarbonyl, cyclopropylcarbonyl, cyclooctylcarbonyl and benzoyl.

The term "lower alkylene" includes C1 to 10, preferably C1 to 6 and more preferably C1 to 3 straight or branched alkylene. Examples are methylene, ethylene, trimethylene, tetramethylene, pentamethylene, hexamethylene, methylmethylene, propylene, dimethylmethylene, 1,1-dimethylethylene and 1,2-dimethylethylene. Methylene, ethylene, dimethylmethylene is especially preferable.

The term "lower alkylene which is optionally intervened by -O-" means alkylene which is the above "alkylene" optionally intervened by 1 to 3 -O-. Alkylene which -O-is intervened at the end is also included. Examples are -O-CH₂ -, -CH₂ -O-, -CH₂ -O-CH₂-, -O-CH₂ -CH₂-, -CH₂ -CH₂ -O-, -O-CH(CH₃)-, -O-C(CH₃)₂ -, -O-CH₂ -CH₂ -O-, -O-CH(CH₃)-O- and -O-C(CH₃)₂ -O-.
"-O-optionally substituted lower alkylene" means alkylene which -O- is intervened at the end.

The term "lower alkenylene" includes C2 to 10, preferably C2 to C6 and more preferably C2 to C4 straight or branched alkenylene having one or more double bond(s) at an arbitrary position(s). Examples are vinylene and propenylene.

The term "halogen" includes fluorine, chlorine, bromine and iodine. Fluorine, chlorine or bromine is especially preferable.

An alkyl part of "lower alkoxy" is the same as the above "lower alkyl".

Examples of the substituent of "optionally substituted lower alkyl", "optionally substituted lower alkylsulfonyl", "optionally substituted lower alkenyl", "optionally substituted lower alkynyl", "optionally substituted lower alkylene", "optionally substituted lower alkenylene", "optionally substituted lower alkoxy" or "optionally substituted acyl" are halogen, hydroxy, optionally substituted lower alkoxy, optionally substituted lower alkynyloxy, optionally substituted amino, mercapto, optionally substituted lower alkylthio, acyl, acyloxy, optionally substituted imino, carboxy, optionally substituted lower alkoxycarbonyl, optionally substituted carbamoyl, optionally substituted thiocarbamoyl, optionally substituted carbamoyloxy, optionally substituted thiocarbamoyloxy, optionally substituted sulfamoyl, optionally substituted lower alkylsulfonyl, optionally substituted lower alkylsulfonyloxy, cyano, nitro, optionally substituted cycloalkyl, optionally substituted cycloalkyloxy, optionally substituted aryl, optionally substituted aryloxy, optionally substituted arylthio, optionally substituted arylsulfonyl, optionally substituted arylsulfonyloxy, optionally substituted heterocycle, optionally substituted heterocycleoxy, optionally substituted lower alkylene, optionally substituted lower alkylenedioxy and oxo. They can be substituted at arbitrary position(s) with at least one group selected from the above. In case that optionally substituted lower alkylene, optionally substituted lower alkylenedioxy is the substituent, the two bonds bind with one carbon atom and taken to form a spiro ring or bind with different atoms and taken together with the neighboring carbon atom to form a ring.
A heterocycle part of "heterocycleoxy" is the same as the above "heterocycle".

Examples of the substituent of "optionally substituted monocyclic aryl", "optionally substituted monocyclic heteroaryl", "optionally substituted aryl", "optionally substituted aralkyl", "optionally substituted aryloxy", "optionally substituted arylthio", "optionally substituted heteroaryl", "optionally substituted hetroaralkyl", "optionally substituted heteroaryloxy", "optionally substituted heteroarylthio", "substituted fused aryl", "substituted fused heteroaryl", "optionally substituted arylsulfonyl", "optionally substituted aromatic carbocyclic diyl" or "optionally substituted aromatic heterocyclediyl" are halogen, hydroxy, optionally substituted lower alkoxy, optionally substituted lower alkynyloxy, optionally substituted amino, mercapto, optionally substituted lower alkylthio, acyl, acyloxy, optionally substituted imino, carboxy, optionally substituted lower alkoxycarbonyl, optionally substituted carbamoyl, optionally substituted thiocarbamoyl, optionally substituted carbamoyloxy, optionally substituted thiocarbamoyloxy, optionally substituted sulfamoyl, optionally substituted lower alkylsulfonyl, optionally substituted lower alkylsulfonyloxy, cyano, nitro, optionally substituted cycloalkyl, optionally substituted cycloalkyloxy, optionally substituted aryl, optionally substituted aryloxy, optionally substituted arylthio, optionally substituted arylsulfonyl, optionally substituted arylsulfonyloxy, optionally substituted heterocycle, optionally substituted heterocycleoxy, optionally substituted lower alkylene and optionally substituted lower alkylenedioxy. They can be substituted at arbitrary position(s) with at least one group selected from the above. In case that optionally substituted lower alkylene or optionally substituted lower alkylenedioxy is the substituent, the bonds can bind with different atoms and taken together with the neighboring carbon atom to form a ring.
Preferable examples in the above substituents are halogen, hydroxy, optionally substituted lower alkyl wherein the substituent is halogen or hydroxy, optionally substituted lower alkenyl wherein the substituent is halogen or hydroxy, optionally substituted lower alkoxy wherein the substituent is halogen or aryl, carboxy, lower alkoxycarbonyl, optionally substituted carbamoyl wherein the substituent is lower alkyl or aryl, optionally substituted amino wherein the substituent is acyl or lower alkyl, mercapto, lower alkylthio, acyl, acyloxy, cyano, nitro, aryl, heterocycle, lower alkylene and lower alkylenedioxy. Halogen or optionally substituted lower alkyl
wherein the substituent is halogen is especially preferable.
A substituent of "substituted benzofuryl", "substituted benzothienyl", "substituted benzopyronyl", "substituted benzoxazolyl", "substituted benzisoxazolyl", "substituted benzothiazolyl", "substituted benzisothiazolyl", "substituted benzimidazolyl" or "substituted benzopyrazolyl" is the same as the substituent of the above "substituted fused heteroaryl".
A substituent of "optionally substituted phenylene" is the same as the substituent of "optionally substituted aromatic carbocyclic diyl".
A substituent of "optionally substituted indolediyl", "optionally substituted benzofurandiyl", "optionally substituted benzothiophenediyl", "optionally substituted furandiyl" or "optionally substituted thiophenediyl" is the same as the substituent of the above "optionally substituted aromatic heterocyclediyl".

Examples of a substituent of "optionally substituted nonaromatic heterocyclediyl" are halogen, hydroxy, optionally substituted lower alkoxy, optionally substituted lower alkynyloxy, optionally substituted amino, mercapto, optionally substituted lower alkylthio, acyl, acyloxy, optionally substituted imino, carboxy, optionally substituted lower alkoxycarbonyl, optionally substituted carbamoyl, optionally substituted thiocarbamoyl, optionally substituted carbamoyloxy, optionally substituted thiocarbamoyloxy, optionally substituted sulfamoyl, optionally substituted lower alkylsulfonyl, optionally substituted lower alkylsulfonyloxy, cyano, nitro, optionally substituted cycloalkyl, optionally substituted cycloalkyloxy, optionally substituted aryl, optionally substituted aryloxy, optionally substituted arylthio, optionally substituted arylsulfonyl, optionally substituted arylsulfonyloxy, optionally substituted heterocycle, optionally substituted heterocycleoxy, optionally substituted lower alkylene, optionally substituted lower alkylenedioxy and oxo. It can be optionally substituted at arbitrary position(s) with at least one group selected from the above. In case that optionally substituted lower alkylene or optionally substituted lower alkylenedioxy is a substituent, the two bonds bind with one carbon atom and taken to form a spiro ring or bind with different atoms and taken together to with the neighboring carbon atom to form a ring.

A substituent of "optionally substituted lower alkynyloxy", "optionally substituted lower alkylthio", "optionally substituted lower alkoxycarbonyl", "optionally substituted lower alkylsulfonyloxy", "optionally substituted cycloalkyl", "optionally substituted cycloalkyloxy", "optionally substituted arylsulfonyl", "optionally substituted arylsulfonyloxy", "optionally substituted heterocycle", "optionally substituted heterocycleoxy" or "optionally substituted lower alkylenedioxy" are the same as the substituent of the above "optionally substituted lower alkyl".

A substituent of "optionally substituted amino", "optionally substituted imino", "optionally substituted carbamoyl", "optionally substituted thiocarbamoyl", "optionally substituted carbamoyloxy", "optionally substituted thiocarbamoyloxy" or "optionally substituted sulfamoyl" is the same as the substituent of the above "optionally substituted lower alkyl". These substituents can be mono- or disubstituted on a nitrogen atom. Lower alkyl, aryl, heterocycle, acyl, lower alkoxycarbonyl, lower alkylsulfonyl or arylsulfonyl is especially preferable.

Preferable embodiments of each sustituent for a compound of the formula (II) are explained below.

Ring Q is monocyclic aryl substituted with at least one of R^{b} and optionally substituted with other group(s), monocyclic heteroaryl substituted with at least one of R^{b} and optionally substituted with other group(s) wherein each R^{b} is optionally substituted aryl, optionally substituted aralkyl , optionally substituted aryloxy, optionally substituted arylthio, optionally substituted heteroaryl, optionally substituted heteroaralkyl, optionally substituted heteroaryloxy or optionally substituted heteroarylthio, substituted fused aryl or substituted fused heteroaryl. Monocyclic heteroaryl substituted with one of R^{b} and optionally substituted with other group(s) wherein R^{b} is optionally substituted aryl, substituted fused aryl or substituted fused heteroaryl is especially preferable.
Examples of substituted fused heteroaryl are substituted benzofuryl, substituted benzothienyl, substituted benzopyronyl, substituted benzoxazolyl, substituted benzisoxazolyl, substituted benzothiazolyl, substituted benzisothiazolyl, substituted benzimidazolyl and substituted benzopyrazolyl. Substituted benzothiazolyl wherein the substituent is halogen, optionally substituted lower alkyl or aryl is especially preferable.

Especially preferable examples of Ring Q are below.
A group of the formula: 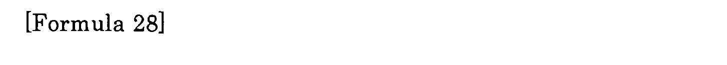 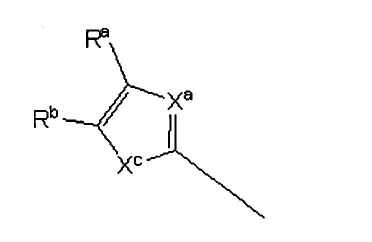 wherein
R^{a} is hydrogen, halogen, hydroxy, optionally substituted lower alkyl, optionally substituted lower alkenyl, optionally substituted lower alkynyl, optionally substituted lower alkoxy or optionally substituted aryl,
R^{b} is optionally substituted aryl, optionally substituted aryloxy, optionally substituted arylthio or optionally substituted heteroaryl, or
R^{a} and R^{b} can be taken together with the neighboring carbon atom to form an optionally substituted ring,
X^{a} is N or CR^{j}, and
X^{c} is NR^{k}, O or S,
wherein R^{j} and R^{k} are each independently hydrogen or optionally substituted lower alkyl or
a group of the formula: 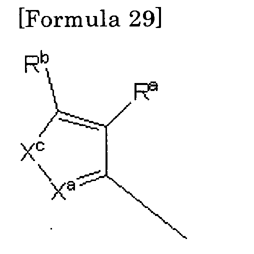 wherein
R^{a} is hydrogen, halogen, hydroxy, optionally substituted lower alkyl, optionally substituted lower alkenyl, optionally substituted lower alkynyl or optionally substituted lower alkoxy,
R^{b} is optionally substituted aryl, optionally substituted aryloxy, optionally substituted arylthio or optionally substituted heteroaryl, or
R^{a} and R^{b} can be taken together with the neighboring carbon atom to form an optionally substituted ring,
X^{a} is N or CR^{L}, and
X^{c} is NR^{m} , O or S,
wherein R^{L} and R^{m} are each independently hydrogen or optionally substituted lower alkyl.

The following embodiments are preferable as "substituted fused heteroaryl" of Ring Q. 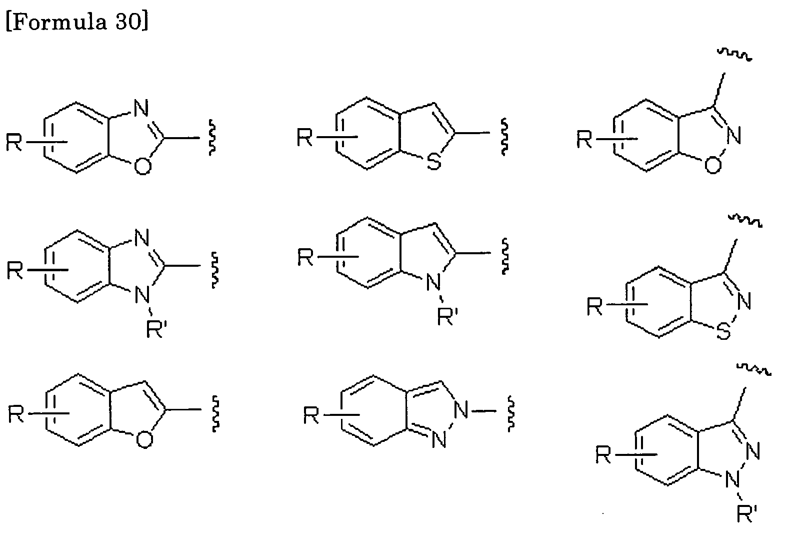 R is halogen, hydroxy, optionally substituted lower alkoxy, optionally substituted lower alkynyloxy, optionally substituted amino, mercapto, optionally substituted lower alkylthio, acyl, acyloxy, optionally substituted imino, carboxy, optionally substituted lower alkoxycarbonyl, optionally substituted carbamoyl, optionally substituted thiocarbamoyl, optionally substituted carbamoyloxy, optionally substituted thiocarbamoyloxy, optionally substituted sulfamoyl, optionally substituted lower alkylsulfonyl, optionally substituted lower alkylsulfonyloxy, cyano, nitro, optionally substituted cycloalkyl, optionally substituted cycloalkyloxy, optionally substituted aryl, optionally substituted aryloxy, optionally substituted arylthio, optionally substituted arylsulfonyl, optionally substituted arylsulfonyloxy, optionally substituted heterocycle, optionally substituted heterocycleoxy, optionally substituted lower alkylene, optionally substituted lower alkylenedioxy or oxo, and
R' is hydrogen or has the same meaning as the above R. Provided that R can be hydrogen when R' has the same meaning as the above R.

The following embodiments are also preferable as "monocyclic heteroaryl substituted with at least one of R^{b} and optionally substituted with other group(s)" for ring Q. 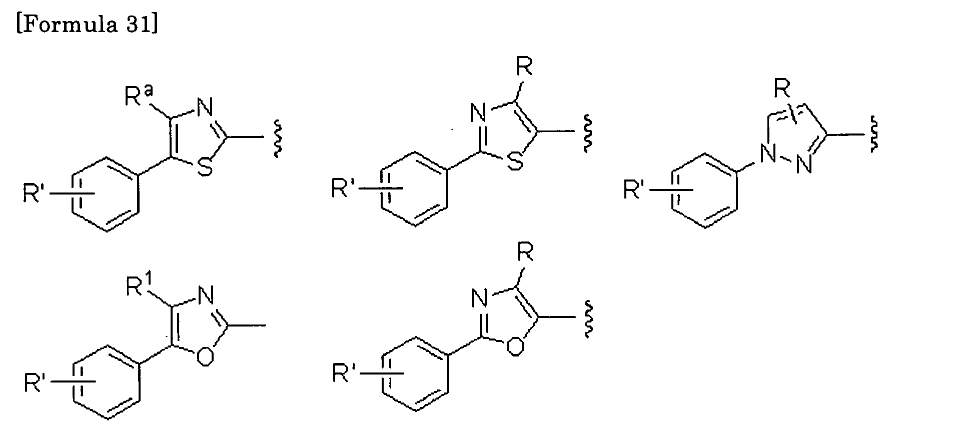 R^{a} is hydrogen, halogen, hydroxy, optionally substituted lower alkyl, optionally substituted lower alkenyl, optionally substituted lower alkynyl, optionally substituted lower alkoxy or optionally substituted aryl, and
R and R' are each independently hydrogen, halogen, hydroxy, optionally substituted lower alkoxy, optionally substituted lower alkynyloxy, optionally substituted amino, mercapto, optionally substituted lower alkylthio, acyl, acyloxy, optionally substituted imino, carboxy, optionally substituted lower alkoxycarbonyl, optionally substituted carbamoyl, optionally substituted thiocarbamoyl, optionally substituted carbamoyloxy, optionally substituted thiocarbamoyloxy, optionally substituted sulfamoyl, optionally substituted lower alkylsulfonyl, optionally substituted lower alkylsulfonyloxy, cyano, nitro, optionally substituted cycloalkyl, optionally substituted cycloalkyloxy, optionally substituted aryl, optionally substituted aryloxy, optionally substituted arylthio, optionally substituted arylsulfonyl, optionally substituted arylsulfonyloxy, optionally substituted heterocycle, optionally substituted heterocycleoxy, optionally substituted lower alkylene, optionally substituted lower alkylenedioxy, oxo or the like.

Y¹ is a bond or -NR^{f} - wherein R^{f} is hydrogen or optionally substituted lower alkyl. A bond is especially preferable.

Ring D is optionally substituted nonaromatic heterocyclediyl, provided that Ring Q binds with a nitrogen atom of Ring D when Y¹ is a bond. Especially preferable group is a group of the formula: 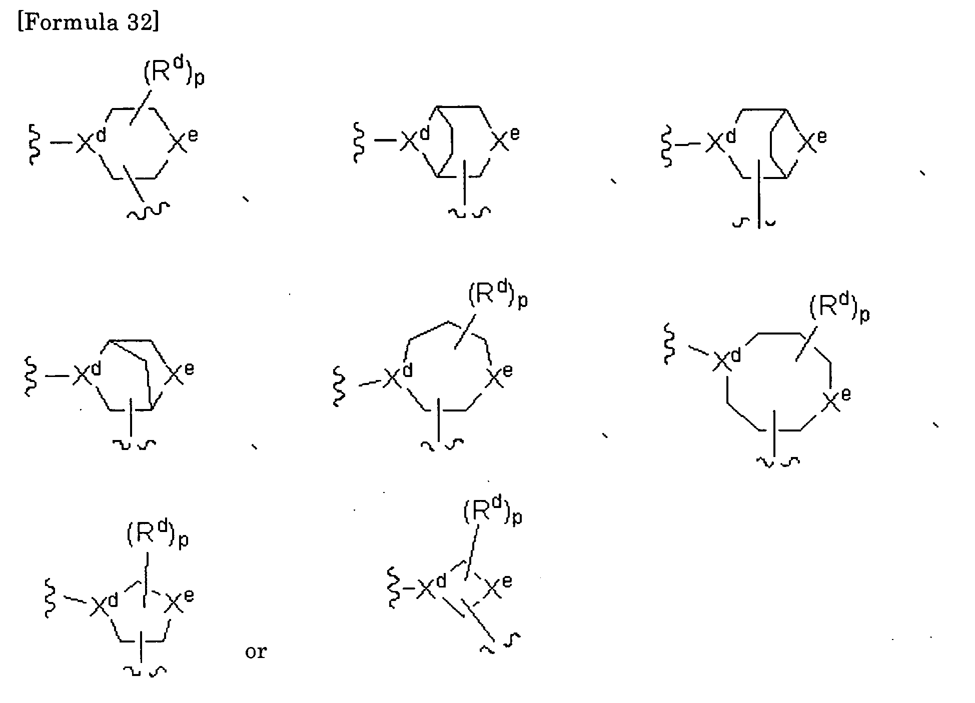 wherein
X^{d} is N or CR^{e} wherein R^{e} is hydrogen or optionally substituted lower alkyl,
X^{e} is O, S, NR^{p} or CR^{q} R^{r} wherein R^{p} to R^{r} are each independently hydrogen or optionally substituted lower alkyl,
provided that
a group wherein X^{d} is CR^{e} and X^{e} is CR^{q} R^{r} is excluded.
R^{d} is each independently, halogen, optionally substituted lower alkyl, optionally substituted lower alkenyl, optionally substituted lower alkynyl, optionally substituted lower alkoxy or optionally substituted aryl, and
p is an integer between 0 and 2,
the bond from X^{d} binds with Y¹ and the other bond binds with Y²,
when X^{e} is NR^{p} or CR^{q} R^{r}, the other bond can bind with X^{e}. A more preferable group is a group of the formula: 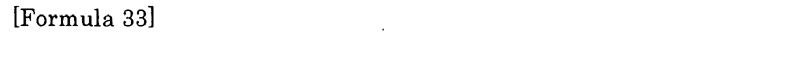 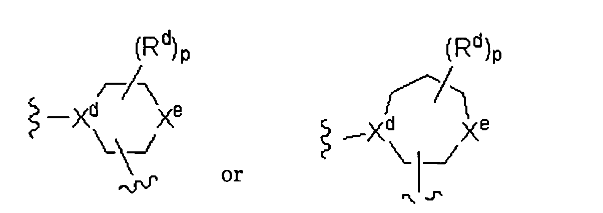 wherein,
X^{d} is N or CR^{e} wherein R^{e} is hydrogen or optionally substituted lower alkyl,
X^{e} is NR^{p} or CR^{q}R^{r} wherein R^{p} to R^{r} are each independently hydrogen or optionally substituted lower alkyl,
provided that,
a group wherein X^{d} is CR^{e} and X^{e} is CR^{q} R^{r} is excluded,
R^{d} are each independently, halogen, optionally substituted lower alkyl, optionally substituted lower alkenyl, optionally substituted lower alkynyl, optionally substituted lower alkoxy or optionally substituted aryl,
p is an integer between 0 and 2,
the bond from X^{d} binds with Y¹ and the other bond binds with Y², and
the other bond can bind with X^{e} . A much more preferable group is a group of the formula: 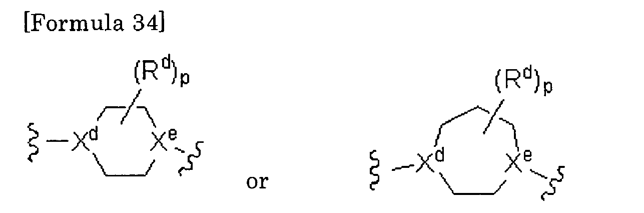 wherein
X^{d} is N,
X^{e} is NR^{p} or CR^{q} R^{r} wherein R^{p} to R^{r} are each independently hydrogen or optionally substituted lower alkyl,
R^{d} are each independently, halogen, optionally substituted lower alkyl, optionally substituted lower alkenyl, optionally substituted lower alkynyl, optionally substituted lower alkoxy or optionally substituted aryl,
p is an integer between 0 and 2,
the bond from X^{d} binds with Y¹ and the other bond binds with Y²,and
a group wherein p is 1 to 2 is especially preferable.

A group of the formula: -Y²Z¹- is a group of the formula: 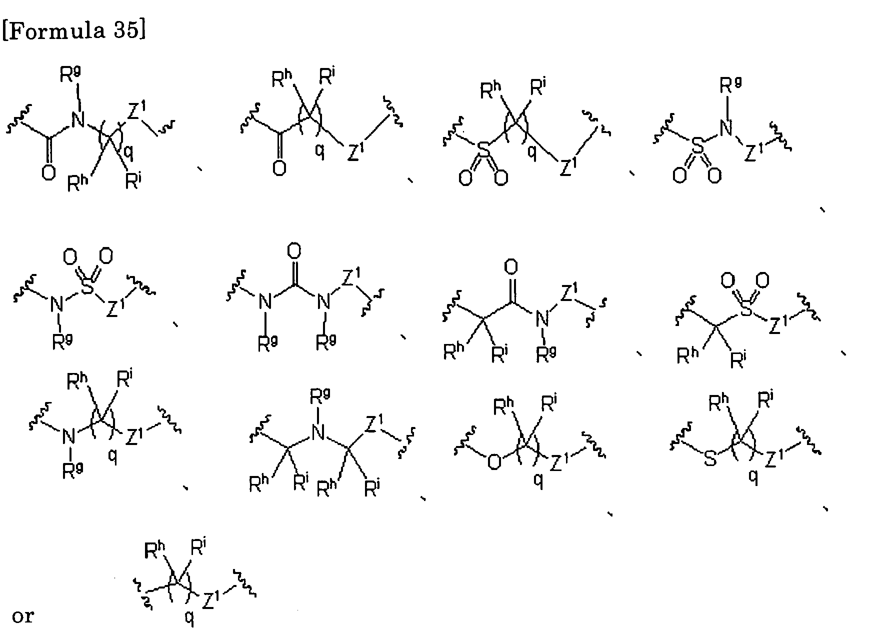 R^{g} are each independently hydrogen or optionally substituted lower alkyl,
R^{h} and Rⁱ are each independently hydrogen or optionally substituted lower alkyl,
q is an integer between 0 and 3, and
Z¹ is a bond, O, S or NRⁱ wherein Rⁱ is hydrogen, optionally substituted lower alkyl, optionally substituted acyl, optionally substituted lower alkylsulfonyl or optionally substituted arylsulfonyl. An especially preferable group is a group of the formula: 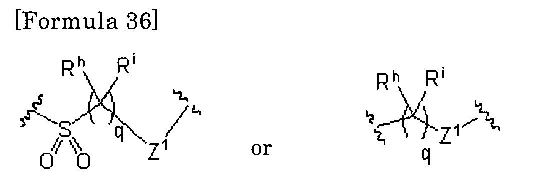 R^{h} and Rⁱ are each independently hydrogen or lower alkyl,
q is an integer between 0 and 2, and
Z¹ is a bond, O or S.

The following embodiments are preferable as a group of the formula: -Y¹-Ring
D-Y²-Z¹-. 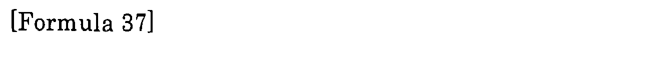 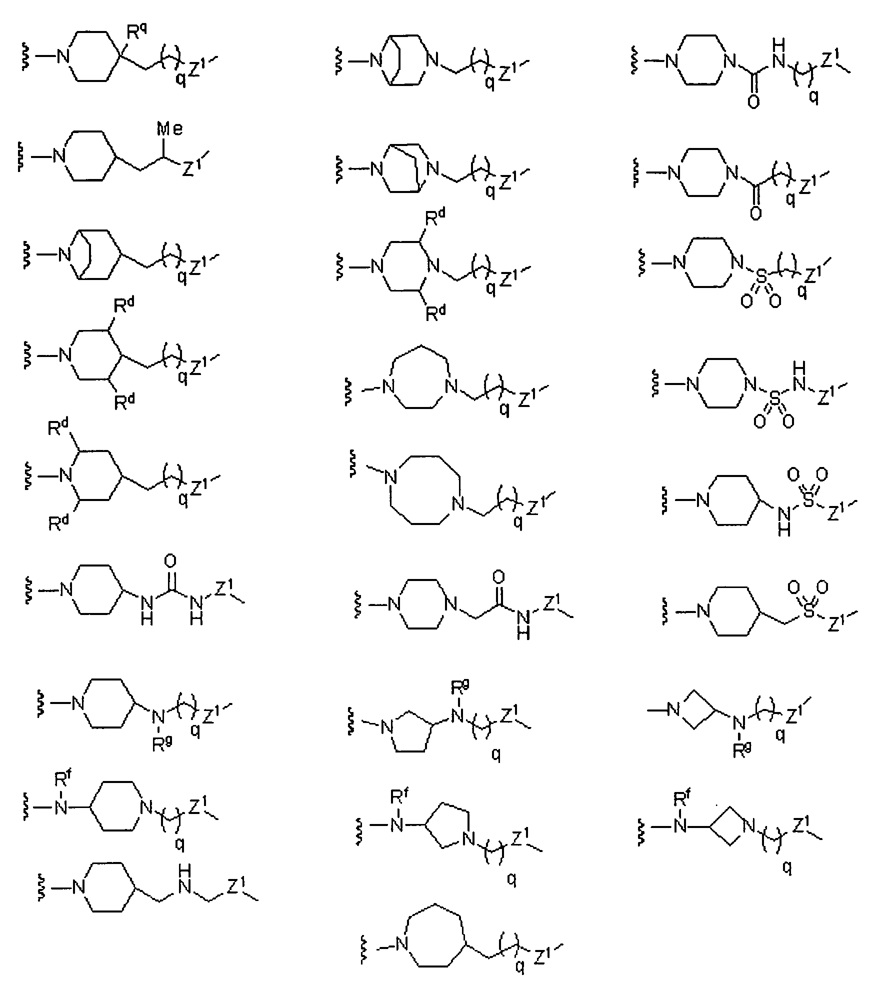 R^{f} is hydrogen or optionally substituted lower alkyl, R^{g} is hydrogen or optionally substituted lower alkyl, R^{d} is each independently halogen, optionally substituted lower alkyl, optionally substituted lower alkenyl, optionally substituted lower alkynyl, optionally substituted lower alkoxy or optionally substituted aryl, Z¹ is a bond, O, S or NRⁱ wherein Rⁱ is hydrogen, optionally substituted lower alkyl, optionally substituted acyl, optionally substituted lower alkylsulfonyl or optionally substituted arylsulfonyl, and q is an integer between 1 and 3.

Ring E is optionally substituted aromatic carbocyclic diyl or optionally substituted aromatic heterocyclediyl. Especially preferable examples are optionally substituted phenylene (the substituent is halogen, lower alkyl or lower alkoxy), optionally substituted furandiyl (the substituent is halogen, lower alkyl or lower alkoxy) and optionally substituted thiophenediyl (the substituent is halogen, lower alkyl or lower alkoxy).

Y³ is a bond, optionally substituted lower alkylene which is optionally intervened by -O- or optionally substituted lower alkenylene. Especially preferable examples are a bond, optionally substituted lower alkylene (the substituent is lower alkylene or halogen), -O- optionally substituted lower alkylene (the substituent is lower alkylene or halogen) and optionally substituted lower alkenylene (the substituent is lower alkylene or halogen).

Z² is COOR^{c}, C(=NR^{c})NRⁿ OR^{o}, CONHCN or a group of the formula: 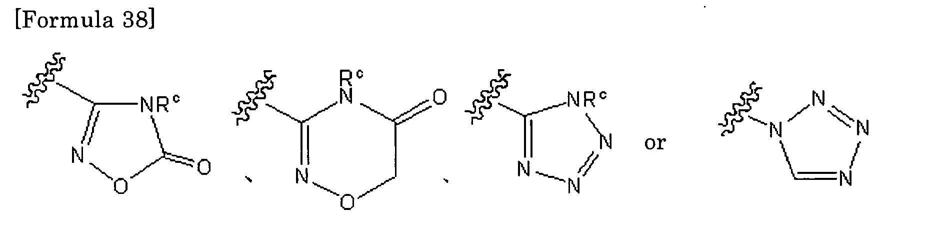 wherein
R^{c}, Rⁿ and R^{o} are each independently hydrogen, optionally substituted lower alkyl, optionally substituted lower alkenyl, optionally substituted aryl or optionally substituted heteroaryl. COOR^{c} wherein R^{c} is hydrogen or optionally substituted lower alkyl, is preferable.

The following embodiments are also preferable as a group of the formula: -Z¹-Ring E-Y³-Z². 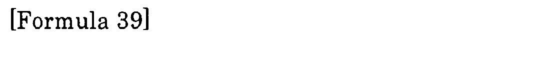 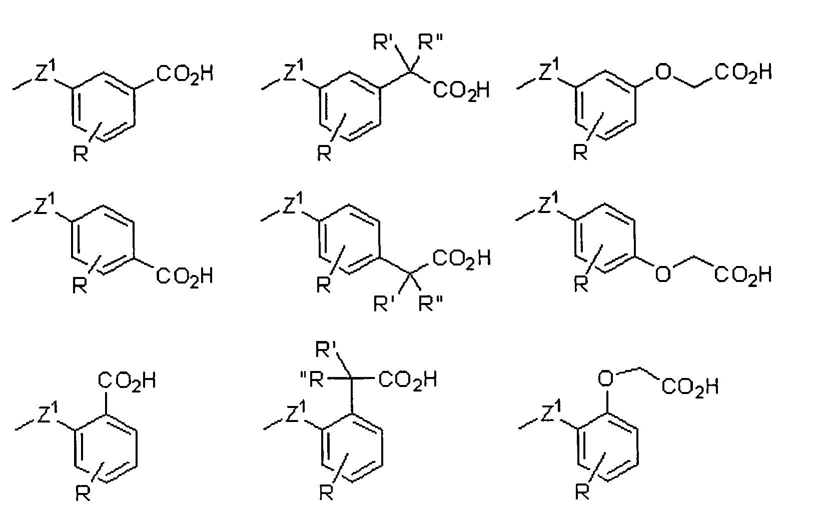 Z¹ is a bond, O, S or NRⁱ wherein NRⁱ is hydrogen, optionally substituted lower alkyl, optionally substituted acyl, optionally substituted lower alkylsulfonyl or optionally substituted arylsulfonyl, and
R, R' and R" are each independently hydrogen, halogen, hydroxy, optionally substituted lower alkoxy, optionally substituted lower alkynyloxy, optionally substituted amino, mercapto, optionally substituted lower alkylthio, acyl, acyloxy, optionally substituted imino, carboxy, optionally substituted lower alkoxycarbonyl, optionally substituted carbamoyl, optionally substituted thiocarbamoyl, optionally substituted carbamoyloxy, optionally substituted thiocarbamoyloxy, optionally substituted sulfamoyl, optionally substituted lower alkylsulfonyl, optionally substituted lower alkylsulfonyloxy, cyano, nitro, optionally substituted cycloalkyl, optionally substituted cycloalkyloxy, optionally substituted aryl, optionally substituted aryloxy, optionally substituted arylthio, optionally substituted arylsulfonyl, optionally substituted arylsulfonyloxy, optionally substituted heterocycle, optionally substituted heterocycleoxy, optionally substituted lower alkylene, optionally substituted lower alkylenedioxy or oxo.

Examples of "pharmaceutically acceptable salt" are salts of inorganic acid such as hydrochloric acid, sulfuric acid, nitric acid or phosphoric acid; salts of organic acid such as para-toluenesulfonic acid, methanesulfonic acid, oxalic acid or citric acid; salts of organic base such as ammonium, trimethylammonium or triethylammonium; salts of alkali metal such as sodium or potassium; salts of alkaline-earth metal such as calcium or magnesium.

"Solvate" can be coordinate with arbitrary number of solvent molecules to Compound (I). Hydrate is preferable.

When Compound (I) has an asymmetric carbon atom, it contains racemic body and all stereoisomers (diastereoisomer, antipode or the like). When Compound (I) of the present invention has a double bond and there is geometrical isomer at a substituent position of double bond, it includes both types of the isomers.

The above compound having PPARδ agonistic activity can be prepared by a method described in scientific literatures, the above patent publications and patent application descriptions.

The following compounds, pharmaceutically acceptable salts or solvates thereof are especially preferable. 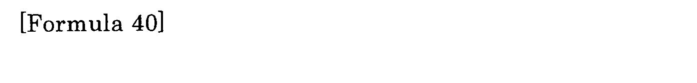 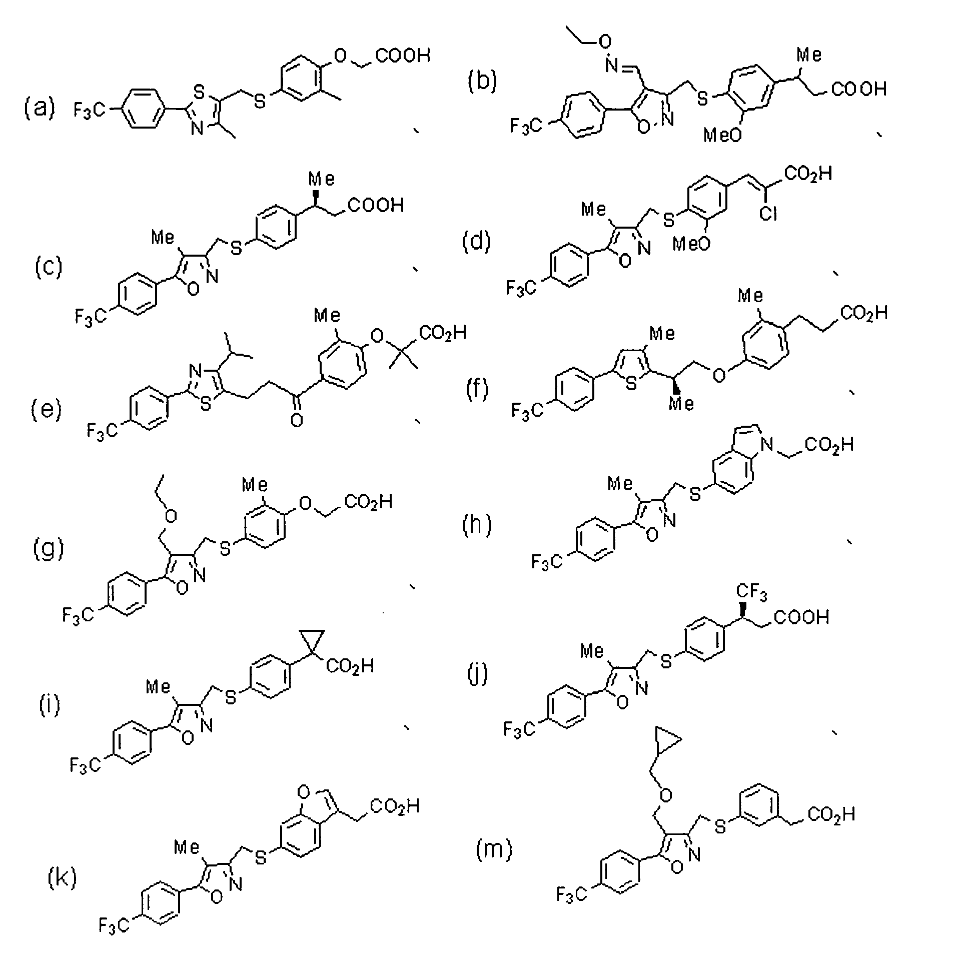 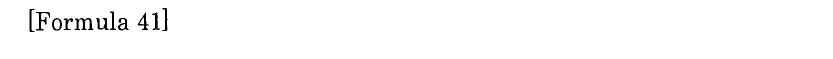 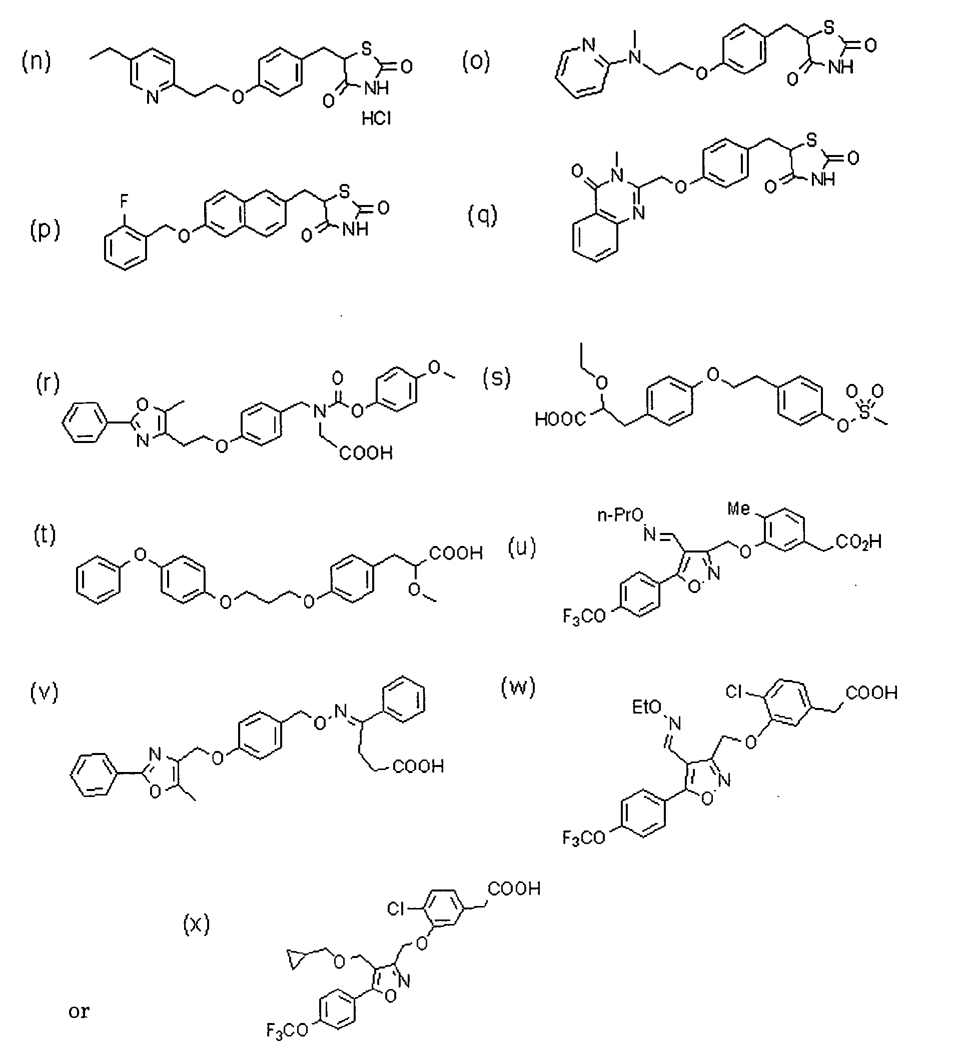 The above compounds having PPARδ agonistic activity used for the present invention can be prepared by methods described in WO01/00603, WO02/014291, WO04/063184, WO2005/054213, Japanese patent application number 2005-155739, PCT/JP2006/310198, US4,687,777, US5,002,953, US5,594,016, WO97/41097 WO01/21602, WO99/62872, WO02/100813, JP2000-34266, US6,166,219, JP1997-295970, EP0745600, US5,886,014 or WO00/71540.
Additionally, a compound having PPARδ agonistic activity can be obtained by measuring the PPARδ agonist activity of a well-known compound. A well-known method can be used as the method for measuring PPARδ activity. For example, it is a method described below.

### Measurement of PPARδ agonistic activity:

A chimeric transcription factor assay, which is commonly used to detect nuclear receptor activity, is employed to measure PPAR transcriptional activity.
Specifically, two plasmids, one that expresses the fusion protein of DNA binding domain of yeast transcription factor GAL4 and a ligand binding domain of a receptor, and a reporter plasmid are transiently transfected to CHO cells. The activity of the promoter containing a recognition sequence of GAL4 coded on the reporter plasmid is used as a parameter to estimate the activity of the receptor.
Plasmid: The ligand binding domain of human PPARδ (hPPARδ) (δ: aa 139 - C-end) is obtained by PCR amplification using Human Universal Quick-Clone cDNA (CLONTECH). Each amplified cDNA is subcloned into pCR2.1-TOPO vector (Invitrogen) and the identity of the cDNA clones is confirmed by the DNA sequence. Then, each obtained cDNA fragment is subcloned into pBIND vector (Promega) to construct a plasmid expressing the fusion protein with DNA binding domain of yeast transcription factor GAL4. pG5luc vector (Promega) is used as a reporter plasmid. Cell culturing and transfection: CHO cells are cultured in 10% FBS-αMEM. With a 96-well plate (Costar), CHO cells, that are dispersed with trypsin treatment, 20000 cells per well and the two plasmids obtained by the above procedure, 25 ng per well, are transfected with FuGene Reagent (Roche) by following the instruction of the manufacture.

Measurement of the transcriptional activity: CHO cells 100 µl per well, which are transfected as above, are dispensed into the wells in which a test compound dissolved in DMSO 0.5 µl is spotted in advance. After the cells and a test compound are cultured together for 24 hours in a CO₂ incubator, the luciferase activity is measured by adding luciferase substrates, PicaGene LT2.0 (Toyo ink) 100 µl per well. LUMINOUS CT-9000D (DIA-IATRON) is used to measure the activity.

As to PPARδ, the concentration of a test compound which shows 1/2 of maximum luciferase activity is calculated using an Excel program, and the EC₅₀ value for PPARδ activity of a test compound is calculated to select a compound whose EC₅₀ value is 0.001 to 1000000(nM) and preferably 0.1 to 100000(nM) as a compound having PPARδ agonistic activity. For example, EC₅₀ value of the above Compound (b) is 1.3 nM. A compound obtained as above can be used as a pharmaceutical combination of the present invention.

A pharmaceutical combination of the present invention is a pharmaceutical combination comprising vitamin K, and
a compound having PPARδ agonistic activity and/or statin compound, in detail,
a pharmaceutical composition comprising a combination preparation or mixture of vitamin K, and
a compound having PPARδ agonistic activity and/or statin compound, or
a kit comprising
vitamin K, and
an agent comprising a compound having PPARδ agonistic activity and/or statin compound.

A pharmaceutical combination of the present invention is not restricted by the quantitative ratio of vitamin K, a compound having PPARδ agonistic activity and/or statin compound. In case of a combination preparation, about 0.01 to 99.9 and preferably 0.05 to 99 weight percent of vitamin K based on weight of a compound having PPARδ agonistic activity and/or statin compound can be contained. In case of a kit, about 0.01 to 99.9 and preferably 0.01 to 99 weight percent of vitamin K based on weight of a compound having PPARδ agonistic activity and/or statin compound can be contained.

The kit is, for example, a therapeutical kit comprising in the same package:
a) a first set of vials for intravenous infusion, said first set of vials comprising a compound having PPARδ agonistic activity and/or statin compound in admixture with pharmacologically acceptable vehicle and/or excipient, a second set of vials for intravenous infusion, said second set of vials comprising vitamin K in admixture with pharmacologically acceptable vehicle and/or excipient;
   or comprising in the same package,
b) a first orally administrable pharmaceutical composition comprising a compound having PPARδ agonistic activity and/or statin compound and a second orally administrable vitamin K in admixture with pharmacologically acceptable vehicle and/or excipient.

In case of a kit, a preparation comprising a compound having PPARδ agonistic activity and/or statin compound can be administered after premedication with vitamin K, or vitamin K can be administered after premedication with a preparation comprising a compound having PPARδ agonistic activity and/or statin compound.

Examples of methods to use a pharmaceutical combination of the present invention are a method of treatment for a disease relating to PPARδ agonistic activity and/or HMG-CoA reductase by coadministeration of vitamin K, a compound having PPARδ agonistic activity and/or statin compound as a composition (In the other words, a method of treatment by giving the above combination preparation), and a method for separately administering them as a part of an appropriate administeration regimen to obtain the benefits of combination therapy (In the other words, a method of treatment with the above kit). An appropriate administeration regimen, dosage for each administeration and each specific administeration interval of each activator depends on a specific combination of used activator, condition of the patient, severity of the condition and the like.

A pharmaceutical combination of the present invention can be administered orally or parenterally. For oral administeration, the pharmaceutical combination of the present invention can be used in any form of usual formulations, for example, tablets, granules, powders, capsules, pills, solutions, syrup, buccals, sublingual tablets or the like which are made by the usual method. For parenteral administeration, the pharmaceutical combination of the present invention can be used in any form of usual formulations, for example, injections such as intramuscular or intravenous administeration, suppository, transdermal therapeutic agent, insufflation or the like. A compound of the present invention can be preferably used as an oral agent because it has high oral bioavailability.

The formulation can be manufactured by combining a curatively effective amount of vitamin K, a compound having PPARδ agonistic activity and/or statin compound with various pharmaceutically acceptable excipients such as binder, moistening agent, disintegrating agents, lubricant, diluent or the like, if necessary. When the formulation is injection, the combination of the present invention may be manufactured by sterilization treatment with an appropriate carrier.

For example, the excipient is lactose, saccharose, glucose, starch, calcium carbonate, crystalline cellulose or the like. The binder is methylcellulose, carboxy methylcellulose, hydroxy propylcellulose, gelatin, polyvinylpyrrolidone or the like. The disintegrating agent is carboxy methyl cellulose, carboxymethylcellulose sodium, starch, sodium alginate, powdered agar, sodium lauryl sulfate or the like. The lubricant is talc, magnesium stearate, macrogol or the like. As a basis for suppository, cocoa butter, macrogol, methylcellulose or the like can be used. When the present invention is manufactured as liquid medicine, emulsion injection or suspension injection, solubilizing agent, suspending agent, emulsifying agent, stabilizing agent, preservatives, isotonic agent or the like which is usually used can be appropriately added. In case of oral administeration, sweetening agent, flavoring agent or the like can be added.

The dose of a pharmaceutical combination of the present invention is preferably established depending on age, body weight, kind of disease, conditions of the patient, the administeration route or the like. In case of the oral administeration for an adult, a compound having PPARδ agonistic activity and/or statin compound which is active ingredient is usually 0.05 to 100 mg/kg/day and preferably 0.1 to 10mg/kg/day. In case of the parenteral administeration, although it is very different depending on route of administeration, a compound having PPARδ agonistic activity and/or statin compound which is active ingredient is usually 0.005 to 10 mg/kg/day and preferably 0.01 to 1mg/kg/day. This can be separated and administrated at 1 time to few times a day.

A pharmaceutical combination of the present invention having PPARδ activity can effectively act for prevention and/or treatment for all diseases concerning PPARδ. As transgenic mice overexpressed PPARδ specifically in fat cells are difficult to get obesity, hyperlipidemia or the like (Cell, Vol. 113, 159-170(2003)), examples of diseases concerning PPARδ are hyperlipidemia, dyslipidosis, disorder of lipid metabolism, Low HDL, High LDL, High VLDL, High TG, diabetes, hyperglycosemia, insulin resistance, obesity, bulimia, arteriosclerosis, atherosclerosis, hypertension, syndrome X, ischemic disease, inflammation, allergic disease (inflammatory bowel disease, rheumatoid arthritis, chronic pancreatitis, multiple sclerosis, glomerulosclerosis, psoriasis, eczema or the like), osteoporosis, sterility, cancer (breast cancer, colonic cancer, colon cancer, ovarian cancer, lung cancer or the like), Alzheimer's disease, Parkinson syndrome or Basedow's disease. The pharmaceutical combination is useful especially for hyperlipidemia, diabetes, disorder of lipid metabolism or the like. Additionally, as compounds having PPARδ selective agonistic activity such as GW501516 (the above Compound (a)) show high elevation of HDL (WO01/00603), the pharmaceutical combination is useful as a HDL enhancer.

Furthermore, a pharmaceutical combination of the present invention comprising a compound having PPARδ activity have inhibiting activity of abnormal blood coagulation and muscular disorder caused by a compound having PPARδ agonistic activity because of vitamin K mixed or used together. The pharmaceutical combination can be a good medicine because the side-effects of the compound having PPARδ agonistic activity can be lightened. Additionally, a pharmaceutical combination of the present invention is further useful when it is used with the other agent causing abnormal blood coagulation and/or vitamin K deficiency symptom such as antibiotics, or the other agent causing muscular disorder such as a statin compound.

A pharmaceutical combination of the present invention comprising a statin compound can effectively act for prevention and/or treatment for all diseases relating to HMG-CoA reductase. Examples of diseases relating to HMG-CoA reductase are hyperlipidemia and hypercholesteremia.

A pharmaceutical combination of the present invention comprising a statin compound have inhibiting activity of muscular disorder caused by a statin compound because of vitamin K mixed or used together. The pharmaceutical combination can be a good medicine because side-effects of the statin compound can be lightened. Additionally, a pharmaceutical combination of the present invention is further useful when it is used with the other agent causing muscular disorder such as fibrate drugs.

The present invention includes a muscular disorder suppressant comprising vitamin K. Then, a pharmaceutical combination of the present invention has inhibiting activity of muscular disorder because of vitamin K mixed or used together. The pharmaceutical combination can be used as a preventive or pharmaceutical agent for muscular disorder caused by, for example, exogenous (crash syndrome), excessive muscular activity, muscular ischemia, metabolic disorder, drug, toxicosis, infectious disease, hereditary disease or heatstroke. Additionally, the pharmaceutical combination can be a good medicine because side-effects of the agent causing muscular disorder such as statin compounds and fibrate drugs can be lightened.

The following examples are provided to explain in more detail and do not restrict the present invention.

### Example 1

5-week-old male Sprague-Dawley rats (Crj:CD(SD)IGS, SPF) were purchased from CHARLES RIVER LABORATORIES JAPAN, INC., Hino Breeding Center.
After holding in a barrier-type animal room to check and quarantine, they housed one animal per cage in plastic cages (clean cage; W262 ×D425 × H150 mm, CLEA Japan, Inc.). They kept in a barrier-type animal room under the condition of a room temperature of 21 to 25°C, a relative humidity of 40 to 70 %, ventilation at 10 times or more/hour with fresh air, and lights on for 12 hours from 8 A.M. to 8 P.M.. CRF-1 pellet food (Selling agency; CLEA Japan, Inc.) sterilized with high pressure steam at 121 °C for 7 minutes was provided as feed. Water irradiated by water sterilizer after filtration with 30 and 3 µm-pore-size filters was provided with an automatic water supplying device as drinking water. The rats were given the feed and water ad libitum.

Compound (a), (b) and (c) having PPARδ agonistic activity (Compound (a) to (c) corresponds to the Compound (a) to (c) described in Claim 10. The activities are shown in Table 1.) were prepared as 3% (w/v) suspension in a vehicle of 0.5% methyl cellulose with mortar and pestle and kept in 4 °C.

**[Table 1]**

| No. | EC₅₀ (nM) |
|---|---|
| | hPPARδ |
| Compound (a) | 3.9 |
| Compound (b) | 1.3 |
| Compound (c) | 18.9 |

Administeration (300mg/kg/day) was started at 7-week-old. The administeration is a 7-day repeated gavage administeration once every morning with injection syringe attached to gastric tube for rats (Fuchigami Medical) at 10mL/kg/day. Dosing volume was calculated based on body weight measured on that day. Controls were administered the vechicle of 0.5 % methyl cellulose by the same manner.

On the next day of the final administeration, rats were opened the abdominal cavity under anesthesia of pentobarbital sodium (Nembutal Injection, Dainippon Pharmaceutical Co., Ltd) and the blood was collected from the posterior vena cava. After collecting, the blood treated with sodium citrate (vacuum blood collection tubes with 3.8% sodium citrate, TERUMO CORPORATION) was separated by centrifugation under cooling at 4 °C, about 1500xg for 15 minutes. With the obtained blood plasma, prothrombin time (PT, second), activated partial thromboplastin time (APTT, second) and fibrinogen concentration (Fbg, mg/dL; Measurement with a calibration curve made by using standard human plasma for blood coagulation test) were measured by CA-6000 Automatic Coagulation Analyzer (SYSMEX CORPORATION) (All of them were measured by a scattered light detection). Thromboplastin · C Plus for PT measurement, DATAFAY ·APTT (Dade activated cephaloplastin reagent) and 20 mM calcium chloride for APTT measurement, DATAFAY FIBRINOGEN THROMBIN REAGENT (Dade thrombin reagent) and Owren's Veronal Buffer (20 mM calcium chloride was derived from Sysmex International Reagents Co., Ltd.. The others were derived from Dade Behring Marburg) for Fbg measurement were used as a reaction reagent.

The results are shown in Table 2. PT and APTT of the rats with Compound (a), (b) and (c) were prolonged more than those of Controls. 3 rats with Compound
(a) died and their samples could not be obtained.

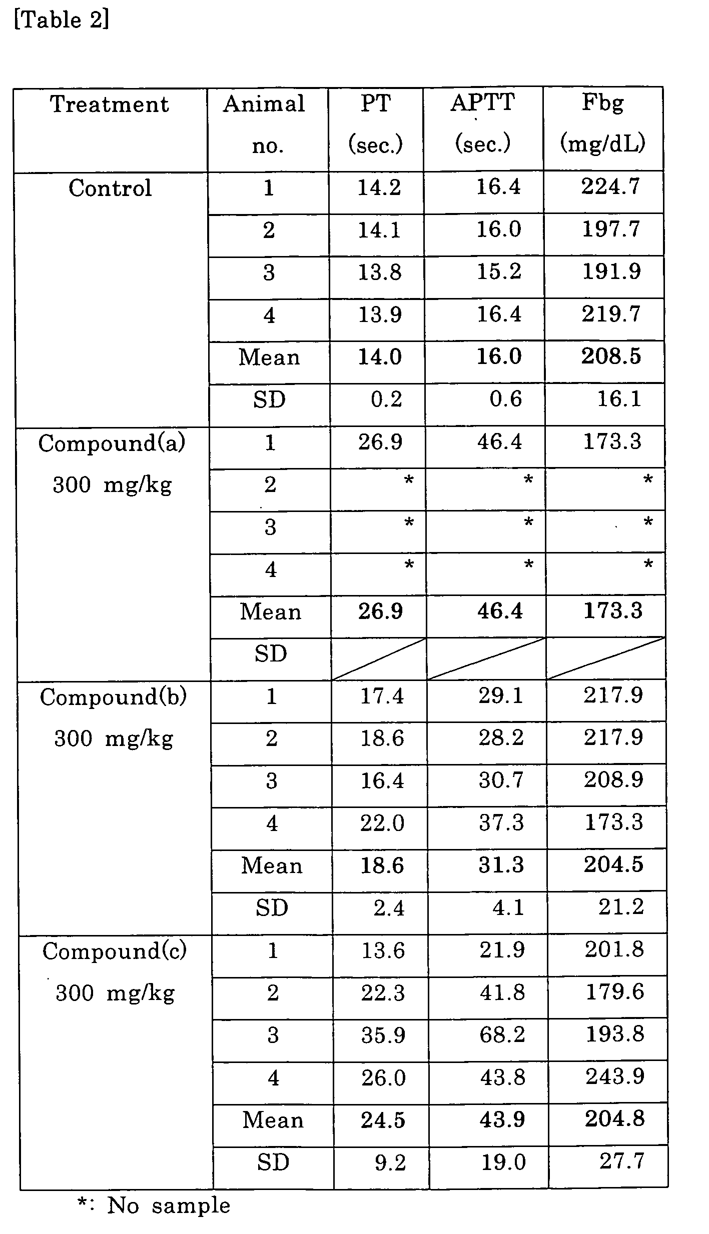

### Example 2

4-week-old male Sprague-Dawley rats (Crj:CD(SD)IGS, SPF) were purchased from CHARLES RIVER LABORATORIES JAPAN, INC., Hino Breeding Center. Methods for animal husbandry, drug preparation and administeration were carried out according to Example 1.

Administeration of Compound (a) (300mg/kg/day) was started at 6-week-old. On the next day of 1, 2 and 3 times administeration, the blood was collected and PT, APTT and Fbg were measured after blood treatment according to Example 1. Additionally, the activities of extrinsic blood coagulation factors (F-III, F-V, F-VII, F-X) and intrinsic blood coagulation factors (F-VIII, F-IX, F-XI, F-XII) were measured. The coagulation times of extrinsic or intrinsic blood coagulation factors were measured by a method for measurement of PT (extrinsic) or APTT (intrinsic) after adding plasma which is deficient a factor of each measuring object (Dade Behring Marburg) to plasma of rat and preincubating. Plasma of 3 control rats was mixed and calibration curve was made with diluent which the plasma was sequentially double-diluted with Owren's Veronal Buffer as a reference material. Activity percentage was calculated with coagulation time measured by a method for coagulation time with factor deficient plasma and calibration curve and activities of each coagulation factor were evaluated by activity percentage compared to plasma of intact rat. The blood was kept under ice-cooling until centrifugation after collecting. In case that the measurement was carried out after the day of collecting, the plasma after separation was kept in a deep freezer (set value:-80 °C).

The results are shown in Table 3. PT and APTT of the group administered twice or more were prolonged more than those of controls. F-II, F-VII, F-X and F-IX, which are vitamin K-dependent coagulation factors, in the once administered group were shown the lower activities than those of controls. The activities of vitamin K-dependent coagulation factors were lowered as times of doses increased.

**[Table 3]**

| Treatment | Times of doses | Animal number | PT (Sec) | APTT (Sec) | Fbg (mg/dL) | F-II (%) | F-V | F-VII (%) | F-X (%) | F-VIII (%) | F-IX (%) | F-XI (%) | F-XII (%) |
|---|---|---|---|---|---|---|---|---|---|---|---|---|---|
| Control | 3 | Mean | 14.5 | 17.8 | 190.8 | 102.5 | 103.3 | 110.8 | 96.5 | 86.7 | 95.8 | 97.0 | 83.1 |
| | | SD | 03 | 10 | 7.4 | 5.8 | 10.3 | 31.2 | 14.0 | 16.3 | 6.2 | 6.0 | 10.5 |
| Compound (a) 300 mg/kg | 1 | Mean | 14.6 | 19.9 | 200.1 | 60.8 | 93.3 | 57.7 | 68.3 | 99.2 | 62.5 | 84.0 | 79.4 |
| | | SD | 09 | 09 | 4,1 | 11 1 | 17.1 | 27.4 | 16.7 | 14.7 | 6.8 | 2.4 | 1.4 |
| | 2 | Mean | 18.3 | 25.5 | 198.8 | 242 | 90.6 | 17.1 | 447 | 942 | 34.2 | 76.0 | 80.7 |
| | | SD | 21 | 26 | 238 | 4.0 | 14.7 | 7.2 | 7.6 | 19.0 | 5.8 | 5.7 | 3.4 |
| | 3 | Mean | 21.7 | 30.7 | 175.7 | 15.1 | 93.0 | 8.8 | 32.4 | 101.2 | 24.6 | 71.0 | 78.6 |
| | | SD | 20 | 1.1 | 159 | 0,5 | 3.9 | 2.7 | 6.1 | 4.2 | 3.0 | 5.6 | 4.6 |

### Example 3

4-week-old male Sprague-Dawley rats (Crj:CD(SD)IGS, SPF) were purchased from CHARLES RIVER LABORATORIES JAPAN, INC., Hino Breeding Center. Animal husbandry and the preparation of Compound (a) having PPARδ agonistic activity were carried out according to Example 1. 0.0003, 0.005 and 0.02 % (v/v) solution of vitamin K1 (TOYO Pharma Co., Ltd., 30mg/3mL) were prepared just before use with a vehicle of water for injection (Otsuka Pharmaceutical Factory, Inc.).

Administeration was started at 6-week-old and Compound (a) (300mg/kg/day) was administered 3 times according to Example 1. Vitamin K1 was administered 1 hour or more after administeration of Compound (a). Subcutaneous administeration was a 3-day administeration (3 or 200µg/kg/day) into the back skin with injection syringe attached with a subcutaneous 1/4 needle at 1 mL/kg/day. Oral administeration was a 3-day repeated gavage administeration (200µg/kg/day) with gastric tube for rats at 4 mL/kg/day. Dosing volume was calculated based on body weight measured on that day. Except for the group of combination administeration, an intact group, a group of only subcutaneous administeration of vitamin K1 of 200µg/kg/day and a group of administeration of Compound (a) of 300mg/kg/day were also set up.

On the next day of the final administeration, the blood was collected and PT, APTT and Fbg were measured after blood treatment according to Example 1. Activities of Vitamin K-dependent coagulation factors (F-II, F-VII, F-X, F-IX) were also measured with the same blood plasma samples according to Example 2.

The results are shown in Table 4. There were no significant differences between each item of the intact group and the group of administeration of vitamin K1 only. As to the group of administeration of Compound (a) only, PT and APTT were more prolonged and the activities of vitamin K-dependent coagulation factors were lowered than the intact group. As to the group of combination administeration of Compound (a) and vitamin K1 of 3 µg/kg/day, prolongation of PT and APTT was reduced and the activities of vitamin K-dependent coagulation factors were higher than a group of administeration of Compound (a) only to show the recovering tendency, but they did not recovered to the level of the intact group. In case of the group of combination administeration of Compound (a) and vitamin K1 of 200µg/kg/day, PT, APTT and activities of vitamin K-dependent coagulation factors were recovered to the level of the intact group in despite of the administeration route (oral or subcutaneous).

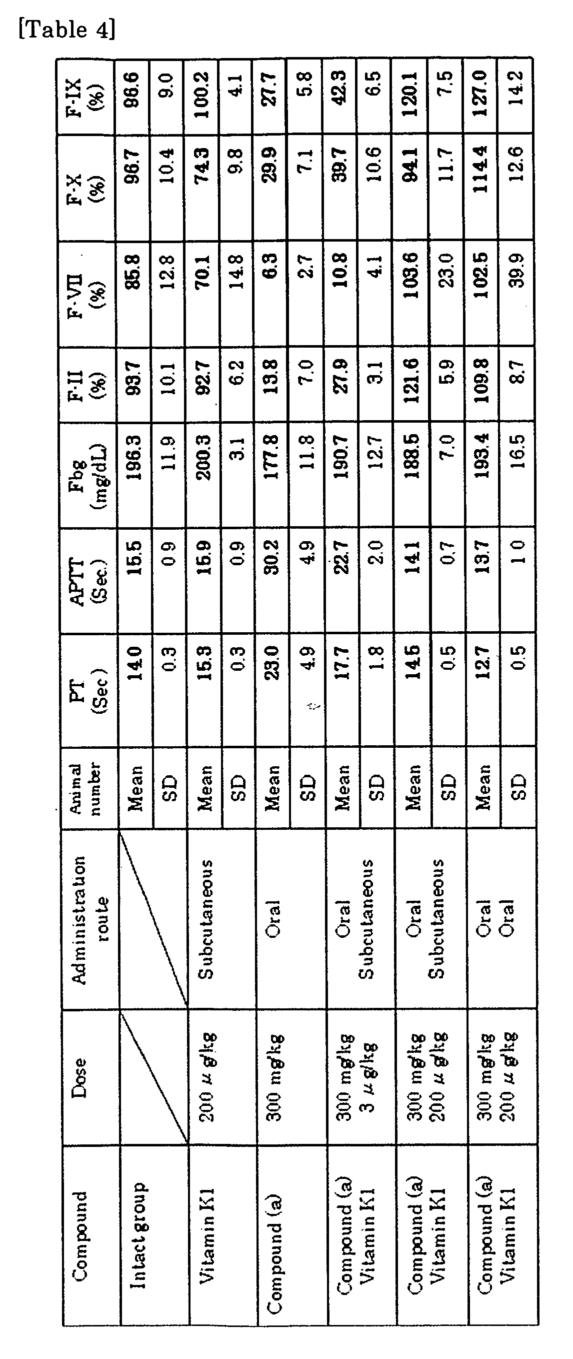

### Example 4

4-week-old male Sprague-Dawley rats (Crl:CD(SD), SPF) were purchased from CHARLES RIVER LABORATORIES JAPAN, INC., Hino Breeding Center. Animal husbandry and the preparation of Compound (a) which is a PPARδ agonist were carried out according to Example 1. 0.005 % (v/v) solution of vitamin K1 (TOYO Pharma Co., Ltd., 30mg/3mL) was prepared just before use with a vehicle of water for injection (Otsuka Pharmaceutical Factory, Inc.).

Administeration was started at 6-week-old and Compound (a) (200mg/kg/day) was administered at 7 times according to Example 1. 15 minutes or more after administration of Compound (a), Vitamin K1 was administered by 7-day repeated gavage administeration (200µg/kg/day) with gastric tube for rats at 4 mL/kg/day. Dosing volume was calculated based on body weight measured on that day. Except for the group of combination administeration, a group of administeration of Compound (a) of 200mg/kg/day was also set up.

On the next day of the final administeration, the blood was collected and prothrombin time (PT), activated partial thromboplastin time (APTT) and fibrinogen concentration (Fbg) were measured after blood treatment according to Example 1. The rats were dissected after collecting the blood and a visual inspection of skeletal muscle (soleus muscle) was carried out. After that, the muscle was fixed with 10% neutral buffered formalin, paraffin section was routinely processed, and a histological examination was carried out with an optical microscope after hematoxylin and eosin staining.

The results are shown in Table 5. As to the group of administeration of Compound (a), prothrombin time and activated partial thromboplastin time were prolonged, necrosis and regeneration, and inflammatory cell infiltration consisted mainly of mononuclear cells in skeletal muscle (soleus muscles of 2/2 rats) were observed. As to the group of combination administeration of Compound (a) and vitamin K1 of 200 µg/kg/day, prothrombin time and activated partial thromboplastin time were recovered to normal values and muscular disorder were not observed.

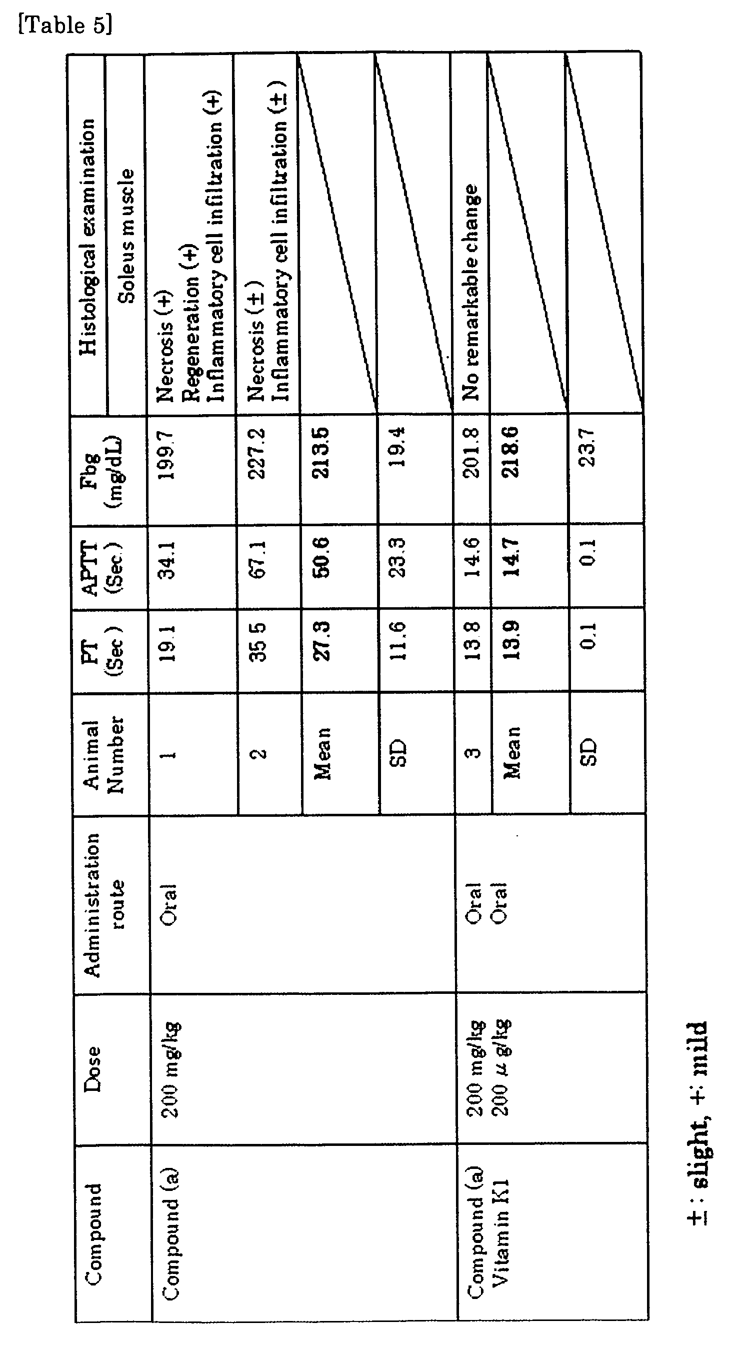

### Industrial Applicability

A pharmaceutical combination of the present invention can suppress abnormal blood coagulation caused by a compound having PPARδ agonistic activity. Additionally, it can suppress muscular disorder caused by a compound having PPARδ agonistic activity, a statin compound or the like.

## Claims

1. A pharmaceutical combination comprising
1) vitamin K, and
2) a compound having PPARδ agonistic activity and/or statin compound.

2. The pharmaceutical combination of Claim 1, wherein the vitamin K is at least one or two selected from the group consisting of vitamin K1, vitamin K2 and vitamin K3.

3. The pharmaceutical combination of Claim 1, wherein the pharmaceutical combination is a combination preparation.

4. The pharmaceutical combination of Claim 1, wherein the pharmaceutical combination is a kit comprising;
an agent comprising vitamin K, and
an agent comprising a compound having PPARδ agonistic activity and/or statin compound.

5. The pharmaceutical combination of Claim 1, which is a HDL enhancer.

6. The pharmaceutical combination of Claim 1, which is an antihyperlipidemic drug.

7. The pharmaceutical combination of Claim 1, which is a muscular disorder suppressant.

8. The pharmaceutical combination of any one of Claims 1 to 7, wherein the compound having PPARδ agonistic activity is a compound of the formula (I): 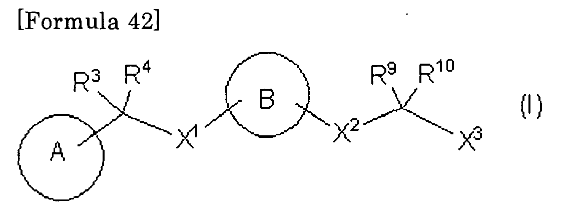 pharmaceutically acceptable salt or solvate thereof,
wherein
Ring A is optionally substituted heteroaryl,
Ring B is optionally substituted aryl or optionally substituted heteroaryl,
R³ and R⁴ are each independently hydrogen, halogen, optionally substituted lower alkyl, optionally substituted lower alkenyl, optionally substituted lower alkynyl, optionally substituted aryl or optionally substituted heterocycle,
R⁹ and R¹⁰ are each independently hydrogen, halogen, cyano, optionally substituted lower alkyl, optionally substituted lower alkoxy, optionally substituted amino or optionally substituted aryl,
X¹ is -O-, -S-, -NR¹¹- wherein R¹¹ is hydrogen, optionally substituted lower alkyl, optionally substituted acyl, optionally substituted lower alkylsulfonyl or optionally substituted arylsulfonyl, -CR¹²R¹³CO-, -(CR¹²R¹³)mO-, -(CR¹²R¹³)mS- or -O(CR¹²R¹³)m-
wherein R¹² and R¹³ are each independently hydrogen or optionally substituted lower alkyl and m is an integer between 1 and 3, -ON=CR¹⁴- wherein R¹⁴ is hydrogen or optionally substituted lower alkyl, or a group of the formula: 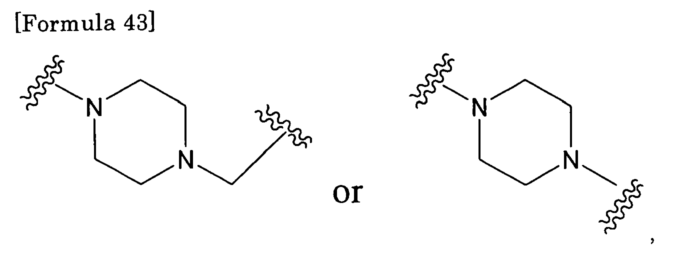 X² is a bond, -O-, -S-, -SO-, -SO₂-, -CR²⁶=CR²⁷- wherein R²⁶ and R²⁷ are each independently hydrogen or optionally substituted lower alkyl, -NR¹⁴- wherein R¹⁴ is hydrogen, optionally substituted lower alkyl, optionally substituted acyl, optionally substituted lower alkylsulfonyl or optionally substituted arylsulfonyl, -CR¹⁵R¹⁶-
wherein R¹⁵ and R¹⁶ are each independently hydrogen or optionally substituted lower alkyl or -COCR²⁴R²⁵- wherein R²⁴ and R²⁵ are each independently hydrogen or optionally substituted lower alkyl,
X³ is COOR¹⁷, C(=NR¹⁷)NR¹⁸OR¹⁹ or a group of the formula: 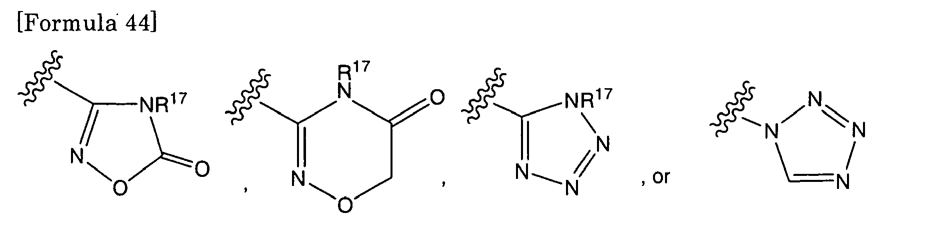 wherein R¹⁷ to R¹⁹ are each independently hydrogen or optionally substituted lower alkyl,
provided that
R⁹ and R¹⁶ can be joined together to form a bond,
R⁹ and R¹⁰ can be taken together to form a ring,
R⁹ and R²⁵ can be joined together to form a bond,
R⁹, R¹⁰ and R¹⁵ can be taken together with the neighboring carbon atom to form a ring,
R¹⁰ and R¹⁵ can be joined together to form a bond, and
R¹⁰ and R¹⁵ can be taken together with the neighboring carbon atom to form a ring.

9. The pharmaceutical combination of any one of Claims 1 to 7, wherein the compound having PPARδ agonistic activity is a compound of the formula (II): 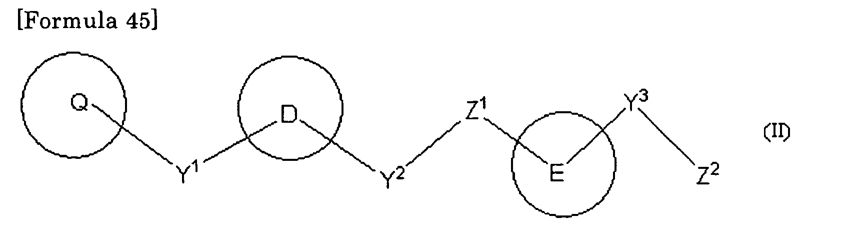 pharmaceutically acceptable salt or solvate thereof,
wherein
Ring Q is monocyclic aryl substituted with at least one of R^{b} and optionally substituted with other group(s), monocyclic heteroaryl substituted with at least one of R^{b} and optionally substituted with other group(s) wherein each R^{b} is optionally substituted aryl, optionally substituted aralkyl, optionally substituted aryloxy, optionally substituted arylthio, optionally substituted heteroaryl, optionally substituted heteroaralkyl, optionally substituted heteroaryloxy or optionally substituted heteroarylthio, substituted fused aryl or substituted fused heteroaryl,
Y¹ is a bond or -NR^{f} - wherein R^{f} is hydrogen or optionally substituted lower alkyl, Ring D is optionally substituted nonaromatic heterocyclediyl, provided that Ring Q binds with a nitrogen atom of Ring D when Y¹ is a bond,
a group of the formula: -Y²Z¹- is a group of the formula: 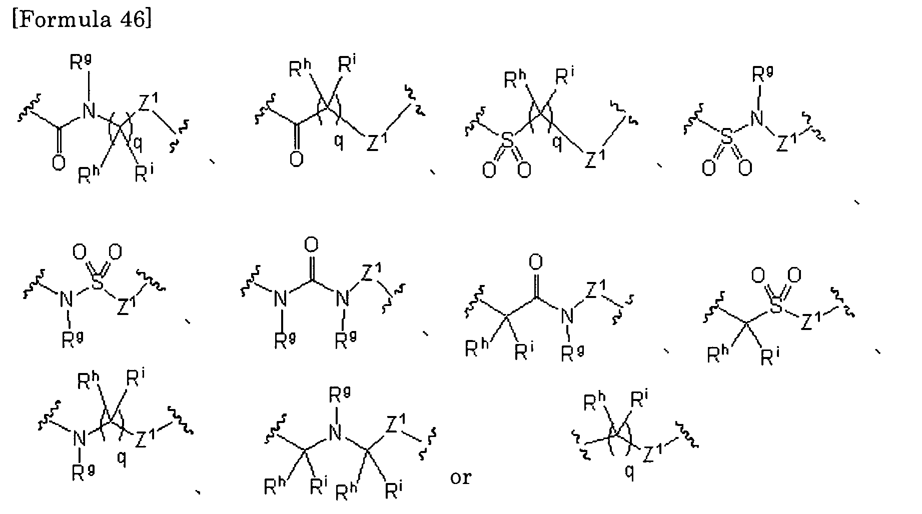 R^{g} are each independently hydrogen or optionally substituted lower alkyl,
R^{h} and Rⁱ are each independently hydrogen or optionally substituted lower alkyl,
q is an integer between 0 and 3,
Z¹ is a bond, O, S or NRⁱ wherein Rⁱ is hydrogen, optionally substituted lower alkyl, optionally substituted acyl, optionally substituted lower alkylsulfonyl or optionally substituted arylsulfonyl,
Ring E is optionally substituted aromatic carbocyclic diyl or optionally substituted aromatic heterocyclediyl,
Y³ is a bond, optionally substituted lower alkylene which is optionally intervened by -O- or optionally substituted lower alkenylene,
Z² is COOR^{c} , C(=NR^{c})NRⁿ OR^{o}, CONHCN or a group of the formula: 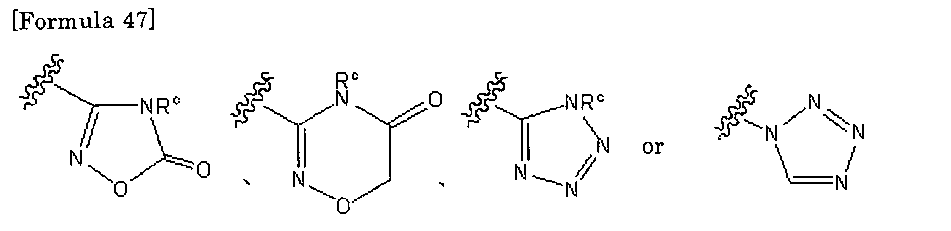 wherein R^{c}, Rⁿ and R^{m} are each independently hydrogen, optionally substituted lower alkyl, optionally substituted lower alkenyl, optionally substituted aryl or optionally substituted heteroaryl,
provided that
a compound wherein a group of the formula: -Y² Z¹- is a group of the formula: 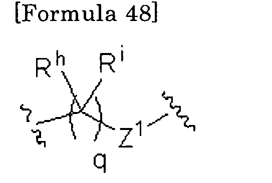 q is 0 and Z¹ is a bond is excluded.

10. The pharmaceutical combination of any one of Claims 1 to 7, wherein the compound having PPARδ agonistic activity is a compound of 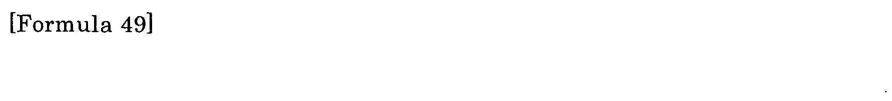 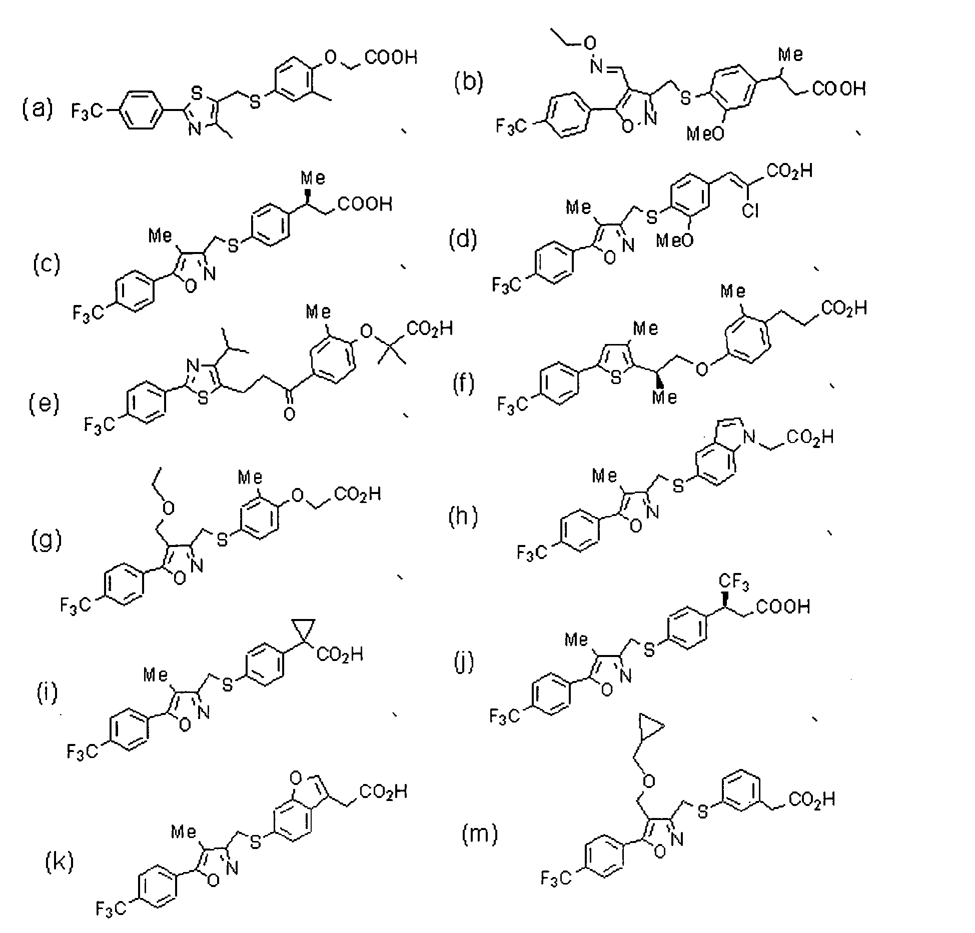 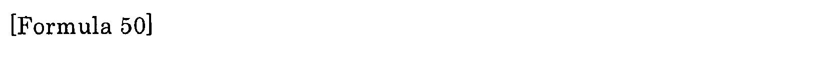 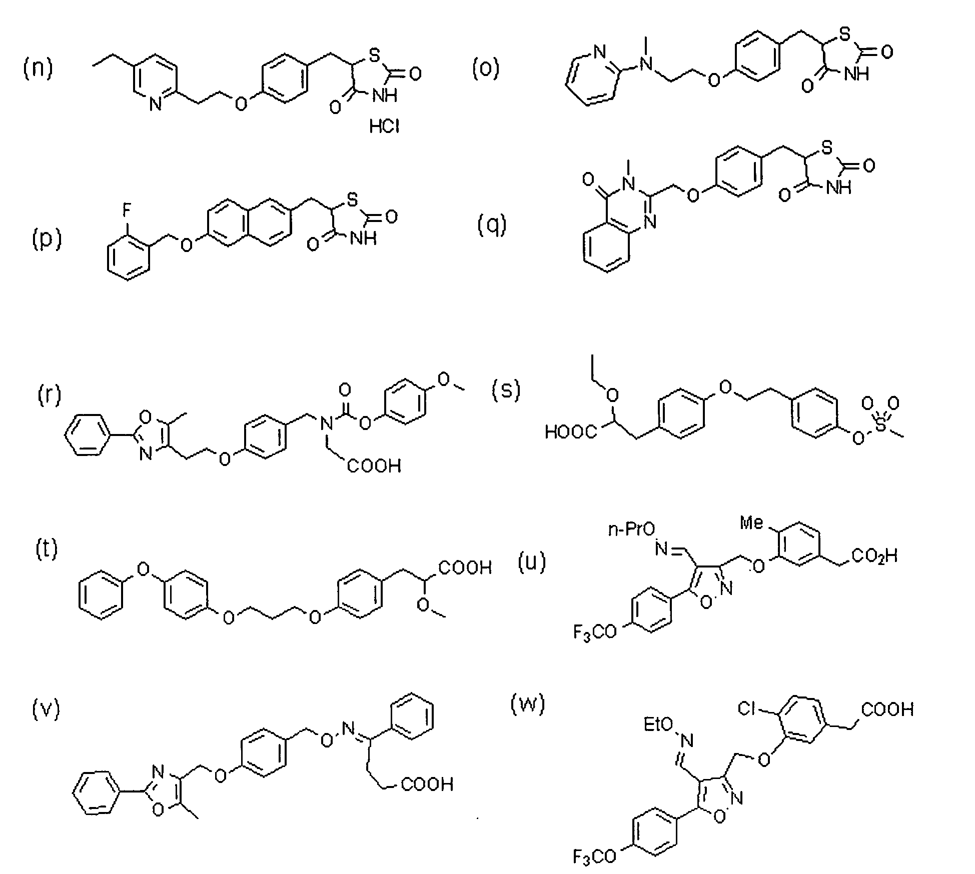 or 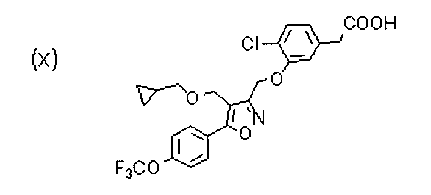 pharmaceutically acceptable salt or solvate thereof.

11. A muscular disorder suppressant comprising vitamin K.
